(19) Europäisches Patentamt
European Patent Office
Office européen des brevets

(11) EP 0 950 709 A1

(12) **EUROPEAN PATENT APPLICATION**

(43) Date of publication:
**20.10.1999 Bulletin 1999/42**

(51) Int. Cl.[6]: **C12N 15/11**, A61K 31/70, C07H 21/04, A61K 47/48

(21) Application number: **98106989.1**

(22) Date of filing: **17.04.1998**

(84) Designated Contracting States:
**AT BE CH CY DE DK ES FI FR GB GR IE IT LI LU MC NL PT SE**
Designated Extension States:
**AL LT LV MK RO SI**

(71) Applicant:
**Hoechst Marion Roussel Deutschland GmbH**
**65929 Frankfurt am Main (DE)**

(72) Inventors:
- **Uhlmann, Eugen, Dr.**
  **D-61479 Glashütten (DE)**
- **Peyman, Anuschirwan, Dr.**
  **D-65779-Kelkheim (DE)**
- **Teti, Anna Maria, Prof.Dr.**
  **IT-00040 Ariccia (Rome) (IT)**
- **Villanova, Ida**
  **IT-65124 Pescara (IT)**

# (54) Antisense oligonucleotides for the inhibition of integrin alphaV-subunit expression

(57) The present invention relates to an oligonucleotide or a derivative thereof which has a sequence that corresponds to a part of a nucleic acid which encodes an integrin $\alpha_V$ subunit and which has the ability to induce apoptosis, the preparation and the use thereof.

EP 0 950 709 A1

## Description

The present invention relates to an oligonucleotide or a derivative thereof which has a sequence that corresponds to a part of a nucleic acid which encodes an integrin $\alpha_v$ subunit and which has the ability to induce apoptosis, the preparation and the use thereof.

The integrin $a_v\beta_3$ is the best known vitronectin receptor, however, it is highly promiscous recognizing RGD in a wide array of adhesive proteins, such as fibronectin, fibrinogen, osteopontin, von Willebrand factor, thrombospondin and collagen [Hynes, Cell 69 (1992) 11-12; Horton, Int J. Biochem. Cell Biol. 29 (1997) 721-725].

Despite its promiscous behaviour, integrin $a_v\beta_3$ is not widely expressed. It is highly expressed on osteoclasts, where it is the dominant integrin. Osteoclasts play a key role in the resorption of bone which leads to osteoporosis. The integrin $a_v\beta_3$ mediates the adhesion of osteoclasts to bone proteins such as osteopontin or bone sialo-protein, thus stimulating bone resorption. [Chorev, Biochemistry 37 (1995) 367-375]. Inhibition of bone resorption in a rat model of osteoporosis has been reported for the systemic administration of a monoclonal antibody [Crippes, Endocrinology 137 (1996) 918-924.] or by the RGD containing snake venom protein echistatin [Fisher, Endocrinology 132 (1993) 1411-1413] as a proof of concept. This makes the integrin $a_v\beta_3$ a promising target for the treatment or prevention of osteoporosis.

Integrin $a_v\beta_3$ is minimally expressed on quiescent blood vessels, but it is significantly upregulated during angiogenesis in vivo [Brooks, Eur. J. Cancer, Part A 32A (1996) 2423-2429; Brooks, DN&P 10 (1997) 456-461]. Angiogenesis plays a role in a variety of pathological states, such as ocular diseases, chronic inflammation, psoriasis, wound healing and cancer. A subsequent blockage of integrin $a_v\beta_3$ function by antibody or peptide antagonist has been shown to efficiently prevent blood vessel formation in several *in vivo* models. [Stroemblad, Chem. Biol. 3 (1996) 881-885]. Preventing integrin $a_v\beta_3$ from binding to its ligands leads to apoptosis selectively in proliferating angiogenic vessels [Brooks, Cell 79 (1994) 1157-1164; Ruoslahti, Kidney Int. 51 (1997) 1413-1417, [Meredith, Trends Cell. Biol. 7 (1997) 146-150]. It seems that integrin $a_v\beta_3$ has a unique function during angiogenesis, namely to provide specific survival signals to facilitate vascular cell proliferation. This function makes integrin $a_v\beta_3$ an interesting target for the therapy of tumors and the other above mentioned diseases.
Restenosis is additional pathological state that can potentially be avoided by targeting integrin $a_v\beta_3$. Smooth muscle cells (SMCs) are another site of integrin $a_v\beta_3$ expression [Hoshiga, Circ. Res. 77 (1995) 1129-1135] and ballon catheder injury induces both osteopontin and integrin $a_v\beta_3$ mRNA expression [Panda, Proc. Natl. Acad. Sci. USA 94 (1997) 9308-9313]. Integrin $a_v\beta_3$ seems to promote the survival and migration of SMCs [Leavesley, J. Cell. Biol. 121 (1993) 163-170], [Clyman, Exp. Cell Res. 200 (1992) 272-284]. The blockage of its activity by peptide antagonists reduces neointimal thickening [Matsuno, Circulation 90 (1994) 2203-2206; Choi, J. Vasc. Surg. 19 (1994) 125-134; Yue, Fundam. Clin. Cardiol. 28 (1997) 69-83].

The mAb LM609 against integrin $a_v\beta_3$ has been used *in vivo* and *in vitro* for the inhibition of angiogenesis in a variety of disease models, such as in a human breast cancer/SCID mouse model [Brooks, J. Clin. Invest. 96 (1995) 1815-1822], in rabbit cormea [Friedlander, Science 270 (1995) 1500-1502], tumor induced angiogenesis on the chick chorioallantoic membrane [Brooks, Cell 79 (1994) 1157-1164]. In summary mAb LM609 leads to a disruption of angiogenesis by inducing apoptosis of angiogenic blood vessels, while no effect is observed on preexisting vessels. This not only prevented the growth of tumors in vivo, but also induced extensive regression in most cases [Brooks, Cell 79 (1994) 1157-1164].

The mAb c7E3 (ReoPro; Centocor/Lilly) which inhibits both integrins $a_v\beta_3$ and $a_{IIb}\beta_3$ recently demonstrated in completed Phase III trials effective reduction in post angioplasty occlusive events, as well as late events associated with restenosis [Topol, Am. J. Cardiol. 75 (1995) 27B-33B].

17E6, an antibody against the integrin $\alpha_v$ subunit, recting with the integrins $\alpha_v\beta_3$, $\alpha_v\beta_5$ and $\alpha_v\beta_1$, strongly inhibited tumor development in nude mouse tumor models [Mitjans, J. Cell. Sci. 108 (1995) 2825-2838].

The cyclic peptide [cyclo-RGDfV] which selectively binds to integrin $\alpha_v\beta_3$ ($IC_{50}$ = 49 nM) (lower case letters indicate D-amino acids) [Pfaff, J. Biol. Chem. 269 (1994) 20233-20238; Wermuth, J. Am. Chem. Soc. 119 (1997) 1328-1335] has also been successfully used by Brooks et al. [Brooks, Cell 79 (1994) 1157-1164] to inhibit tumor induced angiogenesis on the chick chorioallantoic membrane.

Another possibility of inhibition of function of integrin $\alpha_v\beta_3$ is the use of antisense oligonucleotides [E. Uhlmann and A. Peyman, Chemical Reviews 90, 543 (1990); S. Agrawal, TIBTECH 1996, 376]. The synthesis of integrin $\alpha_v$ subunit in melanoma cells was suppressed by a 18-mer integrin $\alpha_v$ subunit all-phosphorothioate antisense oligonucleotide [Nip, J. Clin. Invest. 95 (1995) 2096-2103]. Inhibition of mouse integrin $\alpha_v$ subunit using a mouse-specific 21-mer $\alpha_v$ antisense oligonucleotide [Martin, P. T., & Sanes, J. R. (1997) Development 124, 3909-3917] and a mouse-specific 43-mer $\alpha_v$ antisense oligonucleotide [Wada, J., Kumar, A., Liu, Z., Ruoslahti, E., Reichardt, L., Marvaldi, J., & Kanwar, Y. S. (1996) J. Cell Biol. 132, 1161-76] has also been described. No induction of apoptosis was obesrved on treatment with any of the three described oligonucleotides. In all three cases, the antisense oligonucleotides were used as all-phosphorothiates.

Nip et al described an antisense oligonucleotide AS-3, which is directed against nucleotides 41-58 of the human vitronectin receptor $\alpha_V$ subunit (HSVTNR = human integrin $\alpha_V\beta_3$; AC M14648). This region encompasses the translational start site of HSVNTR.

The present invention provides an oligonucleotide or a derivative thereof which

a) has a sequence that corresponds to a part of a nucleic acid which encodes an integrin $\alpha_V$ subunit and
b) has the ability to induce apoptosis .

The part of the nucleic acid to which the oligonucleotide (hereinafter "ON") corresponds has a length of 5 to 100 nucleotides or more, preferably of 8 to 26 nucleotides, most prefered is a length of 10 to 20 nucleotides. Therefore an oligonucleotide of the invention has a length of 5 to 100 nucleotides or more, preferably of 8 to 26 nucleotides, most prefered is a length of 10 to 20 nucleotides. In special embodiments of the invention the oligonucleotide has a length of 18, 16, 14 or 12 nucleotides.

The oligonucleotide has a sequence that corresponds to a part of a nucleic acid which encodes an integrin $\alpha_V$ subunit. "Corresponds" to means that the order of bases of the oligonucleotide allows the oligonucleotide to hybridize to that part of the nucleic acid to which the oligonucleotide sequence corresponds. With respect to the nucleic acid the order of nucleobases within the oligonucleotide sequence might be the same ("sense oligonucleotide") or the opposite ("antisense oligonucleotide").
Furthermore, the oligonucleotide might have one or more mismatches in comparison to the nucleic acid sequence.

The oligonucleotide might hybridize to mRNA, double and single stranded DNA or cDNA respectively. The oligonucleotide might be an antisense oligonucleotide, a triple helix forming oligonucleotide, a ribozyme or a aptamer. In a prefered embodiment of the invention the oligonucleotide is an antisense oligonucleotide.

The nucleic acid sequence which encodes integrin $\alpha_V$ subunit might be any sequence which encodes the 125 kDa fragment or the 25 kDa fragment of the integrin $\alpha_V$ subunit or a part thereof. The nucleic acid might be a cDNA, mRNA or a gene or a part thereof. The source of the nucleic acid sequence might be any animal, preferably a mammalian animal, most preferably a human. In a prefered embodiment of the invention, the oligonucleotide sequence is deduced from or corresponds to respectively a part of the human integrin $\alpha_V$ subunit cDNA.

The sequence of the human integrin $\alpha_V$ subunit cDNA is available from gene-databases such as e.g. EMBL or NCBI. Human integrin $\alpha_V$ subunit sequences can for example be obtained with the accession numbers M14648, J02826 and M18365.
A part of the human integrin $\alpha_V$ subunit cDNA is available from Suzuki et al. (1986) Proc. Natl. Acad. Sci. USA 83, p. 8616. In a prefered embodiment of the invention the oligonucleotide corresponds to a part of SEQ ID NO. 1 (Table 1).

Within SEQ ID NO. 1 two regions exist: a) core region 1 (SEQ ID NO. 2):
nucleotides 37-60 and core region 2 (SEQ ID: NO. 3): nucleotides 122-147 of the HSVTNR gene shown in SEQ ID NO. 1 (core regions are underlined. In a prefered embodiment of the invention the oligonucleotide corresponds to SEQ ID NO. 2 or SEQ ID NO. 3 or a part thereof. The oligonucleotide directed against one of the core regions has e.g. a length of 8 to 26 nucleotides, preferably 10 to 20 nucleotides. In a special embodiment of the invention the oligonucleotide has a length of 18 nucleotides or less.

SEQ ID NO. 2: 5′ - CGGC GATGGCTTTT CCGCCGCGGC -3′
SEQ ID NO. 3: 5′ - GTGCCGCGC CTTCAACCTA GACGTGG -3′

Examples for sequences for an oligonucleotide are:

SEQ ID NO. 4: 3'-GAAGCCGCTACCGAAAAGGC -5′
SEQ ID NO. 5: 3'-CGCGTGAAGCCGCTACCG -5′
SEQ ID NO. 6: 3'- GCTACCGAAAAGGCGGCG -5′
SEQ ID NO. 7: 3'- GCTGCCGAGAGAGCAACG -5'
SEQ ID NO. 8: 3'-GCGGAAGTTGGATCTGC -5'
SEQ ID NO. 12: 3'- GCGGAAGTTGGACCTGC -5′

In a special embodiment of the invention the oligonucleotide has the sequence SEQ ID NO. 6 or a part thereof or the oligonucleotide sequence is deduced thereof (e.g. it has one or more mismatches).

In another prefered embodiment of the invention the nucleic acid is the cDNA of mouse integrin $\alpha_V$ subunit. The cDNA is e.g. disclosed in Wada et al. (1996) J. Cell Biol. 132, p. 1165. An example for an oligonucleotide sequence is SEQ ID NO. 9.

SEQ ID NO. 9: 3'- GCTACCGACGAGGGCCCG -5'

The invention also relates to derivatives of the oligonucleotides, for example their salts, in particular their physiologically tolerated salts. Salts and phsiologically tolerated salts are described in Remingtons Pharmaceuticals Science.

Subject of the invention are oligonucleotides, in particular antisense oligonucleotides directed against the HSVTNR RNA, which are modified to make them resistant against nucleases with the provisio that these oligonucleotides are not uniformly modified with phosphorothioate internucleotide bridges (all-phosphorothiates) and that they induce apoptosis of the correspondingly treated cells.

An oligonucleotide can, for example, be composed completely of the "natural nucleotides" (comprise the "naturalnucleoside bases") deoxyadenosine phosphate, deoxyguanosine phosphate, deoxyinosine phosphate, deoxycytidine phosphate, uridine phosphate and thymidine phosphate. In other embodiments of the invention, an oligonucleotide can, where appropriate, contain one or more modifications, for example chemical modifications. An oligonucleotide can exhibit several identical and/or different modifications.

Therefore, the present invention in addition relates to an oligonucleotide which has one or more chemical modifications in order to improve its properties. Preferably the oligonucleotide is partially modified. In another embodiment of the invention the oligonucleotide is completely modified, with the provisio that if all phosphodiester bridges are replaced by phosphothioate bridges, this is not the only modification (the oligonucleotide contains another type of modification).

Examples of chemical modifications are known to the skilled person and are described, for example, in E. Uhlmann and A. Peyman, Chemical Reviews 90 (1990) 543 and "Protocols for Oligonucleotides and Analogs" Synthesis and Properties & Synthesis and Analytical Techniques, S. Agrawal, Ed, Humana Press, Totowa, USA 1993 and S.T. Crooke, F. Bennet, Ann. Rev. Pharmacol.Toxicol. 36 (1996) 107-129.

The invention relates to an oligonucleotide which comprises one or more modifications and wherein each modification is independently selected from

a) the replacement of a phosphoric diester bridge located at the 3′ - and/or the 5′ - end of a nucleoside by a modified phosphoric diester bridge,
b) the replacement of phosphoric diester bridge located at the 3′ - and/or the 5′ -end of a nucleoside by a dephospho bridge,
c) the replacement of a sugar phosphate molecule from the sugarphosphate backbone by an other molecule,
d) the replacement of a β-D-2″-deoxyribose by a modified sugar radical,
e) the replacement of a natural nucleoside base by a modified base,
f) the conjugation of the oligonucleotide to a molecule which influences the properties of the oligonucleotide and
g) the introduction of a 3′ -3′ and/or a 5′ -5′ inversion at the 3′ and/or the 5′ end of the oligonucleotide.

More detailed examples for the chemical modification of an oligonucleotide are

a) the replacement of the phosphoric diester bridges, for example with phosphorothioate, phosphorodithioate, $NR^1R^{1'}$-phosphoramidate, boranophosphate, phosphate-($C_1$-$C_{21}$)-O-alkyl ester, phosphate-[($C_6$-$C_{12}$)aryl-(($C_1$-$C_{21}$) -O-alkyl]ester, ($C_1$-$C_8$)alkyl-phosphonate and/or ($C_6$-$C_{12}$)-arylphosphonate bridges,

where $R^1$ and $R^{1'}$ are, independently of each other, hydrogen, ($C_1$-$C_{18}$)-alkyl, ($C_6$-$C_{20}$)-aryl, ($C_6$-$C_{14}$)-aryl-($C_1$-$C_8$)-alkyl, preferably hydrogen, ($C_1$-$C_8$)-alkyl and/or methoxyethyl, preferably hydrogen in particular, ($C_1$-$C_4$)-alkyl and/or methoxyethyl, or
$R^1$ and $R^{1'}$ form, together with the nitrogen atom carrying them, a 5-6-membered heterocyclic ring which can additionally contain a further heteroatom from the group O, S and N;

b) the replacement of the 3'- and/or 5'-phosphoric diester bridges with "dephospho" bridges (described, for example, in Uhlmann, E. and Peyman, A. in "Methods in Molecular Biology", Vol. 20, "Protocols for Oligonucleotides and Analogs", S. Agrawal, Ed., Humana Press, Totowa 1993, Chapter 16, 355ff), for example with formacetal, 3'-thioformacetal, methylhydroxylamine, oxime, methylenedimethylhydrazo, dimethylenesulfone and/or silyl groups;

c) the replacement of the sugar phosphate backbone, for example with "morpholinonucleoside" oligomers (described, for example, in E.P. Stirchak et al., Nucleic Acids Res. 17 (1989) 6129) and/or with polyamide nucleic acids ("PNAs") (described, for example, in P.E. Nielsen et al., Bioconj. Chem. 5 (1994) 3) and/or phosphomonoacidic ester nucleic acids ("PHONAs") (described, for example, in Peyman et al., Angew. Chem. Int. Ed. Engl. 35 (1996) 2632-2638);

d) the replacement of the β-D-2'-deoxyribose units, for example with β-D-ribose, α-D-2'-deoxyribose, L-2'-deoxyribose, 2'-F-2'-deoxyribose, 2'-O-($C_1$-$C_6$)alkyl-ribose, 2'-O-($C_2$-$C_6$)alkenyl-ribose, 2'-[O-($C_1$-$C_6$)alkyl-O-($C_1$-$C_6$)alkyl]-ribose, 2'-$NH_2$-2'-deoxyribose, β-D-xylofuranose, α-arabinofuranose, 2,4-dideoxy-β-D-erythro-hexopyranose, and carbocyclic (described, for example, in Froehler, J. Am. Chem. Soc. 114 (1992) 8320) and/or open-chain sugar analogs (described, for example, in Vandendriessche et al., Tetrahedron 49 (1993) 7223) and/or bicyclosugar analogs (described, for example, in M. Tarkov et al., Helv. Chim. Acta 76 (1993) 481);

e) the modification or replacement of the natural nucleoside bases, for example with 5-(hydroxymethyl)uracil, 5-aminouracil, pseudouracil, dihydrouracil, 5-($C_1$-$C_6$)-alkyl-uracil, 5-($C_2$-$C_6$)-alkenyl-uracil, 5-($C_2$-$C_6$)-alkynyl-uracil, 5($C_1$-$C_6$)-alkyl-cytosine, 5-($C_2$-$C_6$)-alkenyl-cytosine, 5-($C_2$-$C_6$)-alkynyl-cytosine, 5-fluorouracil, 5-fluorocytosine, 5-chlorouracil, 5-chlorocytosine, 5-bromouracil, 5-bromocytosine or 7-deaza-7-substituted purines.

f) The chemical modification of the oligonucleotide furthermore comprises the conjugation of an oligonucleotide with one or more molecules which favorably influences/influence the properties (for example cell penetration, nuclease stability, affinity for the HSVTNR target sequence or improved pharmacokinetics) of the oligonucleotide, e.g. the properties of an antisense oligonucleotide and/or of a triple helix-forming oligonucleotide, and/or attacks/attack the target sequence, while binding and/or crosslinking, when the modified oligonucleotide hybridizes with this sequence (oligonucleotide conjugate). Examples of these conjugates are conjugates with polylysine, with intercalating agents such as pyrene, acridine, phenazine or phenanthridine, with fluorescent compounds such as fluorescein, with crosslinking agents such as psoralen or azidoproflavin, with lipophilic molecules such as ($C_{12}$-$C_{20}$)-alkyl, with lipids such as 1,2-dihexadecyl-rac-glycerol, with steroids such as cholesterol or testosterone, with vitamins such as vitamin E, with poly- or oligoethylene glycol, with ($C_{12}$-$C_{18}$)-alkyl phosphate diesters and/or with O-$CH_2$-CH(OH)-O-($C_{12}$-$C_{18}$)-alkyl. These molecules can be conjugated at the 5' end and/or the 3' end and/or within the sequence, e.g. to a nucleoside base. A special embodiment of the chemical modification relates to the conjugation of the oligonucleotide a) with lipophilic molecules, for example ($C_{12}$-$C_{20}$)-alkyl, b) with steroids such as cholesterol and/or testosterone, c) with poly- and/or oligoethylene glycol, d) with vitamin E, e) with intercalating agents such as pyrene, f) with ($C_{14}$-$C_{18}$)-alkyl phosphate diesters and/or g) with O-$CH_2$-CH(OH)-O-($C_{12}$-$C_{18}$)-alkyl. Processes for preparing an oligonucleotide conjugate are known to the skilled person and are described, for example, in Uhlmann, E. & Peyman, A., Chem. Rev. 90 (1990) 543 and/or M. Manoharan in "Antisense Research and Applications", Crooke and Lebleu, Eds., CRC Press, Boca Raton, 1993, Chapter 17, p. 303ff, and/or EP-A 0 552 766.

g) In other, special embodiments of the invention, the oligonucleotide can exhibit 3'-3' and/or 5'-5' inversions at the 3' and/or the 5' end. This type of chemical modification is known to the skilled person and is described, for example, in M. Koga et al, J. Org. Chem. 56 (1991) 3757.

In a special embodiment of the invention the oligonucleotide corresponds to a part of the nucleic acid sequence near the translational start site (SEQ ID NO. 1, nucleotides 1 to 60) (Tables 1 and 2) which is known to be in many instances an efficient region for antisense oligonucleotides. A number of oligonucleotides which have a sequence that corresponds to a part of SEQ ID NO. 1 and which were additionally modified were synthesiszed and characterized. Surprisingly, it was found that an antisense oligonucleotide against nucleotides 50-67 of HSVTNR (numbers refer to SEQ ID NO. 1, Tables 1 and 2) which is named ON 5543 is more effective for the inhibition of HSVTNR expression than other oligonucleotides which are directed against (or correspond to respectively) nucleotides 44-63 (ON 5541), 41-58 (AS-3 from Nip et al.) and 39-56 (ON 5542).

The sequence of ON 5543 corresponds to nucleotides 50-67, ON 5541 corresponds to nucleotides 44-63, AS-3 (Nip et al) corresponds to nucleotides 41-48 and ON 5542 corresponds to nucleotides 39-56.

The oligonucleotides ON 5543, ON 5541, ON 5542 and AS-3 (Nip et al) are modified. Oligonucleotide AS-3 is an all phosphothioate, all phosphodiester bridges are replaced by phosphothioat bridges. Within the other oligonucleotides only particular positions are modified: The localisation of modification within the sequence is shown (the type of modification: replacement of phosphodiester bridges by thiophosphate bridges):

SEQ ID NO. 4: (ON 5541)

3'-G*A*AGC*C*GC*TAC*C*GAAAAG*G*C -5′

SEQ ID NO. 5: (ON 5542) antisense

3'-C*G*C*GT*GAAG*C*CGC*TA*C*C*G -5′

SEQ ID NO. 6: (ON 5543) antisense

3'-G*C*T*AC*CGAAAAGG*CGG*C*G -5′

* is a phosphorothioate residue.

The invention relates also to oligonucleotides which have modifications at the same positions, but another type of modification.

Other oligonucleotides are used as control oligonucleotides to determine sequence specificity of the above oligonucleotides. For example the oligonucleotides SEQ ID. NO. 10, 11 and 7 are used (control oligonucleotides):

SEQ ID NO. 10: ON 5043 (inverted control of ON 5543)

5'-G*C*T*AC*CGAAAAGG*CGG*C*G-3'

SEQ ID NO. 11: ON 5044 (sense of ON 5543)

5'-C*G*ATGGC*TT*TT*CCGCC*G*C-3'

SEQ ID NO. 7: ON 5045 (mismatch of ON 5543)

3'-G*C*T*GC*CGAGAGAG*CAA*C*G-5'

* is a phosphorothioate residue., mismatches in sequence SEQ ID NO. 7 are underlined.

The observed inhibition with the antisense oligonucleotides is specific for the target protein (target protein is integrin $a_v$ subunit) or the target sequence (a nucleic acid which encodes the integrin $a_v$ subunit) respectively, since only the $a_v$ protein levels were reduced, while $b_3$ and actin protein levels remained unchanged. Thus, theses oligonucleotides specifically inhibit the expression of integrin $a_v$ subunit. Furthermore, also shortened versions of ON 5543 can block or inhibit respectively the expression of the human vitronectin receptor $a_v$ subunit.

In addition, the partially phosphorothioated oligonucleotide ON 5543 showed a higher specificity than the all-phosphorothioates used in previous studies (Nip et al., Martin et al.; Wada et al.).

A second region on the HSVTNR RNA was identified wihtin the coding region, nucleotides against which effective antisense oligonucleotides could be identified. For example, ON 5959 covering nucleotides 128-145 (corresponding to nucleotides 128-145) could efficiently inhibit integrin $a_v$ protein synthesis. This region shows high sequence homology between different species, such as human, chicken and mouse.

| | | | |
|---|---|---|---|
| human | 128 | 5´- CGCCTTCAACCTAGACG -3´ | 145 |
| gallus | | 5´- CGCCTTCAACCTGGACG -3´ | |
| mouse | | 5´- CGCCTTCAACCTGGACG -3´ | |

The corresponding antisense sequence:

The corresponding antisense sequence:

SEQ ID NO. 12:

3'- GCGGAAGTTGGACCTGC -5´

SEQ ID NO. 12: ON 5473 ("consensus-1" antisense)

3'- G*C*G G A A G*T*T G G A C*C*T G*C -5'

SEQ ID NO. 13: ON 5474 (inverted control)

5'- G*C*G G A A G*T*T G G A C*C*T G*C 3'

SEQ ID NO. 14: ON 5475 (sense)

5'- C*G C*C T*T*C A A C*C*T G G A*C*G 3'

SEQ ID NO. 8: ON 5959 ("consensus-2" antisense)

3'- G*C*G G A A G*T*T G G A T*C*T G*C -5'

underlined: 5-propinyl pyrimidines, * phosphothioate bridges

The oligonucleotide "consensus-1" has one mismatch against human, but is perfect against mouse and chicken.
The oligonucleotide "consensus-2" is perfect against human, but has one G-T-mismatch against mouse and chicken.

In a particular embodiment of the invention, an oligonucleotide is prepared by only replacing some of the phosphodiester bridges with phosphorothioate bridges. In particular, the invention comprises oligonucleotides which are only modified to a minimal extent. The principle of minimally modified oligonucleotides is described in A. Peyman, E. Uhlmann, Biol. Chem. Hoppe-Seyler, 377 (1996) 67-70. In this case, 1-5, more preferably 1-3 terminal nucleotide units at the 5' end/or and at the 3' end are protected, for example with phosphorothioates, as are also internal pyrimidine positions. Minimally modified oligonucleotides exhibit particularly advantageous properties; for example they exhibit a particularly high degree of nuclease stability in association with minimal modification.

In a special embodiment of the invention at the 3′ and/or the 5′ end of the oligonucleotide 1-5 nucleotides are modified. In another special embodiment of the invention at least one non-terminal pyrimidine nucleoside and/or a phosphodiester bridge located at the 3′ and/or the 5′ end of that pyrimidine nucleoside is modified.

Special embodiments of the invention include a minimally modified oligonucleotide. For example, a minimally modified oligonucleotide can exhibit the following phosphorothioate patterns:

SEQ ID NO. 6: 5'-G*C*GGC*GGAAAAGC*CA*T*C*G-3'

SEQ ID NO. 8: 5'-C*GT*C*TAGGT*T*GAAGG*C*G -3′

(* denotes phosphorothioate)

These minimally modified oligonucleotides can, for example, also include modifications of the bases, in particular 5-propynylpyrimidines, as well:

SEQ ID NO. 8: 5'-C*GT*C*TAGGT*T*GAAGG*C*G -3′

(C : 5-Propinylcytosin, T : 5-Propinyluracil)

A further prefered embodiment is constituted by chimeric oligonucleotides composed of DNA and 2'-O-methyl-RNA, for example:

SEQ ID NO. '6: 5'-G*C*GGC*GGAAAAGC*CA*T*C*G-3'

SEQ ID NO. 8: 5'-C*GT*C*TAGGT*T*GAAGG*C*G -3

(* denotes phosphorothioate bridges, 2'-O-methyl modified nucleotides are underlined)

A further prefered embodiment is constituted by chimeric oligonucleotides composed of DNA and PNA, for example:

SEQ ID NO. 6: 5'-G*C*GGC*Ggaaaagccatcg-3'

SEQ ID NO. 8: 5'-C*GT*C*TAggttgaaggcg-3'

(* denotes phosphorothioate bridges, the sequence of the PNA moiety is indicated by small letters)

SEQ ID NO. 6: 5'-G*C*GGC*Ggaaaagccatcg-3'

SEQ ID NO. 8: 5'-C*GT*C*TAggttgaaggcg-3'

(* denotes phosphorothioate bridges, the sequence of the PNA part is indicated by small letters, the 2'-O-methyl-modified nucleotides are underlined)

A further preferred embodiment is constituted by antisense oligonucleotides having hexadecyl (C16) residues at the 3' or 5' end, for example:

SEQ ID NO. 6: 5'-C16-G*C*GGC*GGAAAAGC*CA*T*C*G-3'

The oligonucleotides of the invention efficiently inhibit $\alpha_v$ (= integrin $\alpha_v$ subunit) protein synthesis relative to the control oligodesoxynucleotides (ON s). Furthermore, treatment with the oligonucleotides of the invention induced a dose-dependent, substrate-specific reduction of osteoclast adhesion and inhibited bone resorption with a low $IC_{50}$ ($2x10^{-10}\mu M$). The oligonucleotides of the invention caused morphological changes consistent with cell retraction, and induced apoptosis as observed by DNA staining with bis-benzimide, and confirmed by decoration of early-stage fragmented DNA by TUNEL. Surprisingly, the oligonucleotides of the invention stimulated the expression of the cyclin/cyclin-dependent kinase complex inhibitor $p21^{WAF1/CIP1}$, and inhibited that of the cell survival gene, bcl-2. In contrast, the expression of the cell death promoting gene bax remained unchanged. This led to a reduction of the bcl-2/bax ratio which is known to underly the intracellular signal to apoptosis.

The oligonucleotides of the invention were also active in various cancer cells lines. Thus, Oligonucleotide ON 5543 inhibited cell attachment of breast carcinoma MDA-MB231 cells whereas the corresponding sense and mismatch control oligonucleotides had no effect. The cell adhesion was blocked in a dose-dependent manner at 10 nM to 1 $\mu$M antisense oligonucleotide concentrations. Surprisingly, induction of apoptosis was also observed for the cancer cells treated with the oligonucleotides of the invention which makes them useful for tumor therapy.

Therefore, the present invention relates to an oligonucleotide, which when brought into contact with a cell modulates the expression of at least one protein which is involved in apoptosis. In a first aspect, the invention relates to an oligonucleotide which induces an increase in p21 expression, in particular in $p21^{WAF1/CIP1}$ expression. In a second embodiment, the invention relates to an oligonucleotide, which induces a reduction in bcl-2 expression. In a third embodiment, the invention relates to an oligonucleotide, which did not affect the expression of bax. In a another embodiment of the invention, the oligonucleotide induces a reduction in bcl-2 expression, but has no effect on the expression of bax. In this embodiment of the invention the oligonucleotide causes a reduction of the bcl-2/bax ratio; the oligonucleotide causes the intracellular signal to apoptosis.

In another embodiment of the invention the oligonucleotide induces an inhibition of cell adhesion, preferably this effect is dose-dependent. In a prefered embodiment the oligonucleotide inhibits anchorage-dependent growth. In another embodiment of the invention the oligonucleotide induces an inhibition of bone resorption; preferably, this effect is dose-dependent.

The invention further relates to the process for the preparation of an oligonucleotide according to the invention. The process for preparation comprises the chemical synthesis of the oligonucleotide. Preferably the chemical synthesis is a standard methode used for the sythesis of oligonucleotides, e.g. the phoshoamidite methode described in example 1.

The invention further relates to the use of the oligonucleotide as antisense oligonucleotide, as triple-helix forming oligonucleotide, as adaptamer or as ribozyme. Further, the invention relates to the use of an oligonucleotide for inhibiting the expression of integrin $\alpha_V$ subunit, for modulating the expression of at least one protein which is involved in apoptosis, for inducing apoptosis in a cell, for inhibiting cell adhesion and for inhibiting bone resorption. Further for inhibiting angiogenesis and vascularisation, e.g. by inducing apoptosis of angiogenetic blood vessels, in particular if this vascularisation is associated with the growth of tumors and with metastasis of tumors. Therefore, the invention relates to the use of the oligonucleotides for the treatment of tumors and for preventing metastasis.

The invention further relates to a methode of inhibiting the expression of integrin $\alpha_V$ subunit and/or modulating the expression of a protein which is involved in apoptosis and/or inducing apoptosis and/or inhibiting cell adhesion and/or inhibiting bone resporption, wherein an oligonucleotide of the invention is brought into contact with a cell.

The invention furthermore relates to the use of the oligonucleotides for modulating and also totally or partially inhibiting the expression of the $\alpha_V$ protein and/or mutants thereof, for example for totally or partially inhibiting translation. Therefore, the invention relates to a methode, wherein the oligonucleotide is brought into contact with a cell and a methode for introducing the oligonucleotide in a cell. The invention further relates to a methode for hybridizing the oligonucleotide with a target nucleic acid and to a methode of inhibiting the expression of the target nucleic acid.

The invention furthermore relates to the use of the oligonucleotides as pharmaceuticals and to the use of the oligonucleotides for preparing pharmaceuticals. In particular, the oligonucleotides can be used in pharmaceuticals which are employed for preventing and/or treating diseases which are associated with the expression or an overexpression (increased expression) of the integrin $\alpha_V$ subunit.

The invention further relates to a methode for the preparation of a pharmaceutical composition, which comprises mixing an oligonucleotide according to the invention with physiologically acceptable exipient and if appropiate suitable additives and/or auxiliaries.

The invention furthermore relates to the use of the oligonucleotides, or of pharmaceuticals which comprise these oligonucleotides, for treating diseases in which the integrin $\alpha_V$ subunit or its overexpression is the causative factor or is involved.

The invention relates, to the use of the oligonucleotides or a pharmaceutical composition prepared thereof for the treatment and/or prophylaxis of osteoporosis, for the treatment and/or prophylaxis of cardiovascular diseases, such as restenosis, and for the treatment of cancer, e.g. for inhibiting tumor growth and tumor metastasis. In particular, the invention relates to the use of the oligonucleotides for treating cancer or for preventing tumor metastasis or restenosis, or for preparing pharmaceuticals which can be used for treating cancer or for preventing tumor metastasis or restenosis.

The invention furthermore relates to the use for treating cancer or for preventing tumor metastasis or restenosis in combination with known therapeutic methods, for example with methods which are currently employed for treating cancer or for preventing tumor metastasis or restenosis. Preference is given to combination with radiation therapy and known chemotherapeutic agents, such as cisplatin, cyclophosphamide, 5-fluorouracil, adriamycin, daunorubicin or tamoxifen.

The invention furthermore relates to pharmaceutical preparations which comprise oligonucleotides and/or their physiologically tolerated salts in addition to pharmaceutically unobjectionable excipients and/or auxiliary substances.

The oligonucleotides and/or their physiologically tolerated salts can be administered to animals, preferably mammals, and in particular humans, as pharmaceuticals on their own, in mixtures with each other, or in the form of pharmaceutical preparations which permit topical, percutaneous, parenteral or enteral use and which comprise, as the active constituent, an effective dose of at least one oligonucleotide in addition to customary pharmaceutically unobjectionable excipients and auxiliary substances. The preparations normally comprise from about 0.1 to 90% by weight of the therapeutically active compound. In order to treat restenosis, preference is given to a topical use, for example in the form of administration using a catheter. In the case of cancer, preference is given to infusions and oral administration, while in the case of osteoporosis, preference is given to oral administration.

The pharmaceutical products are prepared in a manner known per se (e.g. Remingtons Pharmaceutical Sciences, Mack Publ. Co., Easton, PA), with pharmaceutically inert inorganic and/or organic excipients being used. Lactose, corn starch and/or derivatives thereof, talc, stearic acid and/or its salts, etc. can, for example, be used for preparing pills, tablets, coated tablets and hard gelatin capsules. Examples of excipients for soft gelatin capsules and/or suppositories are fats, waxes, semisolid and liquid polyols, natural and/or hardened oils, etc. Examples of suitable excipients for preparing solutions and/or syrups are water, sucrose, invert sugar, glucose, polyols, etc. Suitable excipients for preparing injection solutions are water, alcohols, glycerol, polyols, vegetable oils, etc. Suitable excipients for microcapsules, implants and/or rods are mixed polymers of glycolic acid and lactic acid. In addition, liposome formulations which are known to the skilled person (N. Weiner, Drug Develop Ind Pharm 15 (1989) 1523; "Liposome Dermatics, Springer Verlag 1992), for example HVJ Liposomes (Hayashi, Gene Therapy 3 (1996) 878) are suitable. Dermal administration can also be effected, for example, using ionophoretic methods and/or by means of electroporation. Furthermore, use can be made of lipofectins and other carrier systems, for example those which are used in gene therapy.

Systems which can be used to introduce oligonucleotides in a highly efficient manner into eukaryotic cells or into the nuclei of eukaryotic cells are particularly suitable.

In addition to the active ingredients and excipients, a pharmaceutical preparation can also comprise additives, such as fillers, extenders, disintegrants, binders, lubricants, wetting agents, stabilizing agents, emulsifiers, preservatives, sweeteners, dyes, flavorings or aromatizing agents, thickeners, diluents or buffering substances, and, in addition, solvents and/or solubilizing agents and/or agents for achieving a slow release effect, and also salts for altering the osmotic pressure, coating agents and/or antioxidants. They may also comprise two or more different oligonucleotides and/or their physiologically tolerated salts and, furthermore, in addition to at least one oligonucleotide, one or more different therapeutically active ingredients. The dose can vary within wide limits and is to be adjusted to the individual circumstances in each individual case.

The invention relates to a pharmaceutical composition which comprises at least one oligonucleotide according to the invention that can be used for the treatment of diseases which are assoziated with abnormal cell proliferation, cell migration, cell differentiation, angiogenesis, retinal neurite outgrowth, bone resorption, phagocytosis, immune response, signal transduction and the metastasis of neoplastic cells. Such pharmaceutical composition can be used for the treatment and prevention of cancer and metastasis of cancer, the treatment and prevention of osteoporosis, the treatment of ocular diseases, chronic inflammation, psorasis, restenosis and the support of wound healing.

Adhesion of cells to substrate has been recognized as a critical event requiring expression of specific receptors capable of recognizing and binding components of the extracellular matrices, the so-called 'cell adhesion molecules' (1). The integrin receptors represent the most relevant cell-surface protein by which cells bind to extracellular matrices. Permutations of the $\alpha$ and $\beta$ integrin subunits serve as receptors for many adhesive proteins, their interaction with the extracellular matrices being responsible for correct cell growth, survival and function (2) (3). Osteoclasts are bone resorbing, multinucleated cells usually found in contact with the mineralized bone matrix within lacunae which are the result of their resorptive activity (4). Bone resorption requires the osteoclast to attach to the bone surface and firmly adhere to it to form the sealing membrane, a specialized adhesion area which critically contribute to the maintenance of the controlled composition of the resorption lacuna milieu (5). In this extracellular sealed space, proteolytic enzymes and hydrogen ions degrade both the organic and the inorganic components of bone (6). Due to the importance of the sealing membrane organization to osteoclast function, conditions that interfere with the adhesion properties of the cell modulate the resorptive activity (7).

The vitronectin receptor (integrin $a_vb_3$) is present in selected cell types, including osteoclasts where it is highly expressed and plays a functional role related to the resorbing activity (8). The receptor consists of the $\alpha_v$ subunit of ~150kDa, and the $\beta_3$ subunit of ~95 to 115 kDa (9). Besides vitronectin, $a_vb_3$ recognizes the Arg-Gly-Asp (RGD) adhesive sequence motif present in the bone matrix proteins osteopontin and bone sialoprotein. (10,11). As for many $\alpha$ subunits, the $\alpha_v$ chain has a major role in determining the ligand specificity (10,12). Besides the $\beta_3$ chain it can form heterodimers with at least four other b subunits ($\beta_1$, $\beta_5$, $\beta_6$, and $\beta_8$) and has been demonstrated to have several highly conserved regions among species (12). Taken together, this information suggests that the $\alpha_v$ integrin subunit may represent an important target for regulation of specific functions associated with the adhesive capacity of cells.

Strategies aiming at the inhibition of $\alpha_v$-mediated cell adhesion may greatly facilitate therapeutical approaches to osteopenic syndromes, as well as providing insights into the mechanisms underlying impairment of osteoclast activity in osteopetroses. Antisense oligodeoxynucleotides (ON s) offer the opportunities for sequence-specific inhibition of gene expression (13-20). Phosphodiester bonded ON s are rapidly degraded in serum and within cells, with half lives of less than two hours (14, 15,21,22). However, chemical modification of the naturally occurring phosphodiester bonds, such as replacement of a non-bridging oxygen by sulfur, increases their intracellular stability against nucleases (23,24). It was shown (25) that partial phosphorothioation (that is, the modification of internal pyrimidines and the terminal three 5′ and 3′ bases) is equally effective at reducing the amount of target protein and has the additional advantage of decreasing non sequence-related side effects occasionally associated with phosphorothioated molecules (19). In this study, partially phosphorothyoated $\alpha_v$-antisense ON s were used and evidence was obtained that this reagent impairs osteoclast adhesion and bone resorption. Furthermore, this is the first time that $a_v$ antisense ON treatment induces apoptosis of rabbit osteoclasts *in vitro*, and that this apoptotic process is accompanied by altered expression of $p21^{WAF1/CIP1}$ and bcl-2 genes.

Integrin-mediated cell anchorage regulates a number of cell functions (1-3), and the $a_vb_3$ receptor is known to play a critical role in the control of osteoclast activity (38,39). Surprisingly it was found that specific inhibition of $a_v$ integrin expression by an antisense reagent, which reduced both osteoclast adhesion and bone resorption *in vitro*, induced programmed cell death by mechanisms involving the regulation of the $p21^{WAF1/CIP1}$ and the bcl-2, but not the bax genes.

Impairment of adhesion has long been demonstrated to represent an important mechanism for inhibiting bone resorption. In this study it was shown that such inhibition is obtained when the $\alpha_v$ integrin subunit synthesis is potently reduced by an antisense mechanism while the $b_3$ integrin subunit levels remain unchanged. This effect is based on the specificity of Watson-Crick base pairing between the antisense ON and the target mRNA (14,19,22,24,40), which offer the potential to block the expression of specific genes within cells. This has been reported in numerous tissue culture experiments, and in several recent *in vivo* studies (41). Although the mechanism of action of antisense ON s is generally hard to prove (14, 16,42,43), in this study reasonable evidence was obtained supporting a specific antisense effect. Firstly, the observed inhibition is sequence-specific in that only the $a_v$-antisense ON is effective, whereas the sense, inverted and mismatch control ON s were not active. Secondly, the observed inhibition is specific for the target protein, since only the $a_v$ protein levels were reduced, while $b_3$ and actin remain unchanged.

The $a_v$ antisense ON used in this study spanned the AUG start site, was located between bases 220 and 237 of the human sequence and complemented the rabbit gene. This reagent was found to have a high potency, at least in part determined by a local concentrating effect via adsorption to the substrate, which was greater than that of the AS3 and AS4 $a_v$ antisense ON s reported to inhibit melanoma cell adhesion *in vitro* (37).

Antisense DNA approaches have been used to control a number of osteoclast functions (32,44-47). ON s against the 16 kD and 60 kD subunits of the V-ATPase have been demonstrated to inhibit at high doses (approximately 30 mM) rat osteoclast bone resorption and polarization (44,45), shown microscopically by disruption of the F-actin ring structure that is typically observed in resorbing osteoclasts (2,48). The 5543 $a_v$ antisense ON showed no obvious effects on the F-actin ring structures (not shown), but clearly induced programmed cell death, an event which has been demonstrated to rapidly disrupt the cells (49,50).

Adhesion to the substrate has long been considered critical to survival of cells which grow adherent to an extracellular matrix (3). Anchorage-dependent growth has been demonstrated in a variety of cell types to modulate function and gene expression (51,52). The transition from anchorage-dependent to anchorage-independent growth is a characteristic feature of neoplastic transformation, normal cells being dependent upon interaction with the substrate for physiological behavior. Apoptosis is a highly conserved active cellular mechanism characterized by cell shrinkage, chromatin condensation and nuclear fragmentation (49). All these events have been observed in our cells treated with the $a_v$ antisense ON , thus suggesting that alteration of the interaction with the substrate may control the size of the osteoclast population *in vitro*.

Programmed cell death is regulated by a number of genes which control the balance of cell proliferation and cell death (49,50). A cell undergoes apoptosis as a result of information received by the microenvironment and interpreted by the intracellular machinery. Cell-surface receptor-mediated mechanisms which control apoptosis often act through signal transduction systems involving the activation of second messengers regulating the transcription of specific genes (49,50). For example, the $p21^{WAF1/CIP1}$ gene encodes for a protein which inhibits the cyclin/cyclin-dependent kinase, CDK (53). Ligation of $a_vb_3$ during angiogenesis has been demonstrated to suppress the expression of $p21^{WAF1/CIP1}$ and to stimulate cell survival (54). In our study we found that inhibition of $a_v$ integrin synthesis by the 5543 antisense ON stimulated the expression of $p21^{WAF1/CIP1}$ in a mixed population enriched in osteoclasts and osteoclast precursors. This gene was clearly detectable in untreated osteoclasts, as expected for post-mitotic cells, as well as in a small number (9%) of mononuclear cells. However, its immunodetection in osteoclasts was enhanced and, at the same time, the number of mononuclear cells expressing the gene was found to be increased in $a_v$ antisense- relative to control ON -treated cultures. Inhibition of cyclin-CDK complex by $p21^{WAF1/CIP1}$ interferes with phosphorylation events critical to cell cycle transition (53). It has recently been suggested that this cell cycle inhibitor is implicated in growth arrest associated with terminal differentiation (55). Furthermore, potential roles of this gene in events leading to efficient repair of DNA damage or to apoptosis (56,57) has been hypothesized. Expression of the $p21^{WAF/CIP1}$ protein has been found to be suppressed in endothelial cells during $a_vb_3$-mediated adhesion both *in vivo* and *in vitro* (52), and stimulated in response to disruption of fibroblast-to-extracellular matrix interaction (58). These data indicate a potential direct link between integrin-dependent anchorage and regulation of $p21^{WAF1/CIP1}$ to promote cell survival. Among the numerous genes that have been implicated in the control of cell survival, the bcl-2 gene, which encodes two splice variants, Bcl-2a and Bcl-2b, is known to be a negative regulator of cell death (49,50). The bcl-2 prolonges the survival of non-cycling cells, and prevents the death by apoptosis of cycling cells (49,50). In contrast, the bax gene encodes a protein that has a role in opposition to Bcl-2, acting as a death promoting factor. Bcl-2 is known to potentiate cell survival based on its ability to dimerize with Bax (49,50); therefore a high bcl-2/bax ratio promotes cell survival, whereas a low bcl-2/bax ratio induces apoptosis. This study clearly demonstrates that osteoclast programmed death induced by impairment of $a_v$-dependent anchorage, is associated with a dramatic reduction of the bcl-2 expression rather than with changes in bax levels. These events lead to a clear lowering of the bcl-2/bax ratio which is likely to induce apoptosis in osteoclasts and their putative precursors. In this context it is interesting to note that Strömblad et al. (54) recently demonstrated that denied attachment of endothelial cells on a bovine serum albumin-coated surface affected expression of both genes, inducing a decrease of bcl-2 and an increase of bax relative to cells attached to immobilized anti-$a_vb_3$ antibody. These findings indicate that inhibition of $a_v$ integrin dependent anchorage in different cellular models may induce distinct effects on the expression of the bcl-2 and the bax gene products which, lead to a reduction of the bcl-2/bax ratio.

In conclusion, this study provides evidence that an antisense ON targeted to the $a_v$ integrin subunit mRNA of osteoclasts successfully inhibits adhesion and bone resorption with high specificity and potency, and that the $a_v$-dependent anchorage is mandatory for the survival of osteoclasts and their putative precursors. A clear link between

inhibition of $a_v$-mediated adhesion and regulation of $p21^{WAF/CIP1}$ and bcl-2/bax ratio has been demonstrated to represent the underlying pathway that promotes apoptosis in the rabbit osteoclast lineage. The development of this approach opens an avenue to alternative therapies for bone diseases.

Examples:

Abbreviations:

| | |
|---|---|
| BSA: | Bovine Serum Albumin. |
| DMEM: | Dulbecco's modified Minimum Essential Medium. |
| ECL: | Enhanced ChemiLuminescence. |
| FBS: | Fetal Bovine Serum. |
| ON : | oligodeoxynucleotides. |
| PBS | : Phosphate Buffer Saline. |
| TRAP: | Tartrate-Resistant Acid Phosphatase. |
| TUNEL: | TdT-mediated dUTP-biotin nick end labeling. |

Methods

Reagents.

Cell culture media, reagents and sera were from Hyclone (Röntegenstraat, Holland) or Gibco Lifesciences (Inchinnan, UK). Culture dishes and sterile glass ware were from Falcon Becton-Dickinson (Lincoln Park, NJ) and from Costar Co. (Cambridge, MA). Apoptosis/DNA fragmentation detection kit (TUNEL) was from Chemicon (Tamecula, CA). Chemicals, of the purest grade, were from Sigma Chemical Co. (St. Louis, MO). The human $\alpha_v\beta_3$ 23C6 monoclonal antibody (26) was produced in our laboratory. The human $\alpha_v$ monoclonal antibody (clone #139) and the human $\beta_3$ polyclonal antibody (clone #1264) (27), were obtained from the Department of Dermatology, State University of New York at Stony Brook, New York. The anti-$p21^{WAF1/CIP1}$ (Ab-5) antibody was from Oncogene Research Calbiochem (Cambridge, MA). The anti-Bcl-2 (sc 492), the anti-Bax (sc 493), the anti-actin (sc 1616) and the secondary antibodies were from Santa Cruz Biotechnology Inc. (Heidelberg, Germany). ECL kit was from Amersham International plc (Little Chalfont, U.K.). 1,25-dihydroxy vitamin $D_3$ was obtained from Roche S.P.A. (Milan, Italy).

RT-PCR:

Total RNA was prepared from rabbit spleen and bone marrow, high $\alpha_v$ expressing tissues. The two flanking primers, $a_{v1}$ and $\alpha_{v3}$, amplified a predicted band of 198 bp (see Fig. 1). The internal $\alpha_{v2.1}$ and the 3'$\alpha_{v3}$ primers amplify a band of 133 bp, though this product would not include the ATG start site. Therefore, nested PCR using the $\alpha_{v1}$ + $\alpha_{v3}$ product as template, utilizing the primers $\alpha_{v1}$ and $\alpha_{v2}$, were used to amplify an 82 bp band which should include the start site sequence. This was T:A subcloned into pCR2.1 and sequenced automatically in the forward and reverse directions.

Cells:

Freshly isolated osteoclasts were obtained from newborn (4-7 days) New Zealand White rabbits as described by Chambers et al. (30) and Caselli et al. (31). Cells were allowed to attach to substrates for 45-90 min, then incubated in DMEM supplemented with 10% FBS, 100 IU/ml penicillin, 100 μg/ml streptomycin, in a water-saturated atmosphere of 95% air and 5% $CO_2$.
Rabbit osteoclasts were also differentiated *in vitro* from bone marrow. Bone marrow was flushed from long bones and cultured as described above. After 24 hours, non-adherent cells were removed by aspiration and repeated washes, and the total adherent cell fraction was incubated with 10 nM 1,25-dihydroxy vitamin $D_3$ for ten days. At the end of incubation, contaminating stromal cells were removed by mild trypsinization and osteoclast phenotype was evaluated by positive staining for TRAP activity, retraction in response to 100 n M salmon calcitonin, and resorption pit formation.
Mouse osteoclasts were generated *in vitro* and characterized as described by Tanaka et al. (32).

Substrates:

Osteoclasts were plated either onto glass, bone or dentine. 10% FBS-containing DMEM was used as a standard adhesion substrate during cell culture on artificial substrates. Alternatively, glass coverslips were previously coated with adhesive proteins and cells were then incubated in low serum (1%)-containing DMEM. For substrate coating, 20% FBS or 10μg/ml fibrinogen, fibronectin, vitronectin and fibrillar collagen were used as sources of adhesive substrates. Wells were coated with the proteins and incubated 3 hours or overnight at 4°C. The solutions were then replaced with 60% methanol, and the wells were further incubated for 1 h at 4°C. Methanol was removed by aspiration, and the wells were incubated with 100 μl of a buffer containing 50 mM Tris-HCl (pH 7.8), 110 mM NaCl, 5 mM $CaCl_2$, 1% BSA, and 0.1 mM phenylmethylsulfonylfluoride for 30 min at room temperature. Finally, wells were washed three times with DMEM supplemented with 0.2% BSA and immediately used for the experiment.

Treatment with ON s:

ON s were dissolved in water and diluted in DMEM as stock solutions and stored in aliquots at -20°C until used. Treatment of osteoclasts was performed by incubating the cells with appropriate concentrations of the ON s for different periods.

FITC-ON labeled analysis:

FITC-labeled ON were synthesized in order to monitor their uptake into the cells and adsorbance to substrates. Cells, were incubated for 24 hours with 1μM FITC-ON s, fixed and observed by confocal fluorescence microscopy (Leica Tcs-NT system). For adsorption to glass or dentine, ranging concentrations of FITC-ON (0.2-20 μM) were incubated together for up to 24 hours.

Apoptosis:

Cell morphology was evaluated by phase contrast microscopy. Bisbenzimide, which specifically binds to the Adenine-Thymidine regions of DNA, was used for nuclear staining. Cells were fixed in Carnoy's fixative (methanol-glacial acetic acid 3:1), incubated for 30 min in 0.5 μg/ml bisbenzimide, rinsed (2x in distilled water), mounted in glycerol-PBS 1:1 and observed by conventional epifluorescence microscopy.
To evaluate fragmented DNA the TUNEL method (34) was used according to manufacturer's instruction. Briefly, cells were fixed in 4% buffered paraformaldehyde, washed (2 min, 4x in distilled water) and incubated in TdT buffer for 5-10 min at room temperature. Buffer was then removed, and cells were incubated in TdT- and Biotin-dUPT-containing buffer for 60 min at 37°C. Cells were then washed in TdT buffer for 15 min at room temperature, washed (2 min, 4x in distilled water), blocked in blocking reagent and incubated with avidin-conjugated FITC for 30 min at 37°C. Cells were washed in PBS (5 min, 3x), counterstained with 0.5 μg/ml propidium iodide, washed in PBS (3 min), mounted and observed by conventional epifluorescence microscopy.

Statistics:

Data are the mean+SE from at least three independent experiments. Statistical analysis was performed by one-way analysis of variance (ANOVA) followed by the Student's *t* test and by the Mann-Whitney test. A $p$ value < 0.05 was considered statistically significant.

Example 1: Oligodeoxynucleotide synthesis.

The $\alpha_v$ ON sequences and the rabbit AUG start site sequence. The sequences of the ON s used in this study are shown in Table 4. Partially phosphorothioated antisense ON s directed to the $\alpha_v$ subunit of human vitronectin receptor were chosen on the basis of the BLAST alignment program (35). Alignment of the known species variants for the $\alpha_v$ subunit revealed high homologies around the translational start site (Fig. 1A).
Since rabbit osteoclasts were used in the following studies, the rabbit translational start sequence was determined by RT-PCR. Three pairs of degenerate primers were designed, that when used in combination, reveal the rabbit sequence (Fig. 1B and C). Fig. 1D represents the rabbit $\alpha_v$ translational start sequence, including theON 5543 antisense sequence. The sequences are highly homologous (98.9%) with only one base discrepancy at position 83 where the rabbit $\alpha_v$ contained a thymidine base compared with a cytosine in the human sequence (36).
ON s were synthesized using an Applied Biosystems 394 DNA synthesizer (Perkin Elmer Applied Biosystems, Inc., Foster City, USA) and standard phosphoramidite chemistry. After coupling, phosphorothioate linkages were introduced by sulfurization using the Beaucage reagent (28) followed by capping with acetic anhydride and *N*-methylimidazole. After cleavage from the solid support and final deprotection by treatment with concentrated ammonia, ON s were purified by polyacrylamide gel electrophoresis. The *C*-5 propynyl pyrimidine modified ON s were prepared as described previously (29). All ON s were analyzed by negative ion electrospray mass spectroscopy (Fisons Bio-Q) which in all cases confirmed the calculated mass. The C16-modified oligonucleotides were synthesized using hexadecyloxy (cyanoethoxy) N,N-diisopropyl aminophosphane as phosphitylating reagent in the last step of oligonucleotide synthesis in place of a standard amidite. Analysis of the oligonucleotides was done by

a) Analytical gel electrophoresis in 20% acrylamide, 8M urea, 45μM tris-borate buffer, pH 7.0 and/or
b) HPLC-analysis: Waters GenPak FAXcolumn, gradient $CH_3CN$ (400ml), $H_2O$ (1.61), $NaH_2PO_4$ (3.1g), NaCl (11.7g), pH6.8 (0.1M an NaCl) after $CH_3CN$ (400ml), $H_2O$ (1.6l), $NaH_2PO_4$ (3.1g), NaCl (175.3g), pH6.8 (1.5M an NaCl) and/or
c) capillary electrophoresis using a Beckmann capillary eCAP™, U100P Gel Column, 65 cm length, 100 mm I.D., window 15 cm from one end, buffer 140 μM Tris, 360mM borate, 7M urea and/or
d) negativ ion electrospray mass spectrometry which in all cases confirmed the expected mass values.

Following oligonucleotides against (human) HSVTNR mRNA were prepared:

ON 5541 (antisense)

3'-G*A*AGC*C*GC*TAC*C*GAAAAG*G*C- 5'

ON 5542 (antisense)

3'-C*G*C*GT*GAAG*C*CGC*TA*C*C*G- 5'

ON 5543 antisense

3'-G*C*T*AC*CGAAAAGG*CGG*C*G - 5'

ON 5043 (inverted contr. of ON 5543)

5'-G*C*T*AC*CGAAAAGG*CGG*C*G-3'

ON 5044 (sense of ON 5543)

5'-C*G*ATGGC*TT*TT*CCGCC*G*C-3'

ON 5045 (mismatch of ON 5543)

3'-G*C*T*GC*CGAGAGAG*CAA*C*G-5'

ON 5959 antisense

5'- C*G T*C*T A G G T*T*G A A G G*C*G -3'

ON 5972 inverted Control

5'- G*C*G G A A G*T*T G G A T*C*T G*C -3'

ON 5970 sense

5'-C*G C*C T*T*C A A C*C*T A G A*C*G -3'

ON 5971 mismatch

5'- C*G T*C*T G A G T*T*G A G A G*C*G -3'

ON 5431 antisense

3'- G*C*T*A C* C G A A A A G G*C G G*C*G - C16 -5'

ON 5432 inverted repeat

5'- G*C*T*A C* C G A A A A G G*C G G*C*G - C16 -3'

ON 5504 antisense

3'- G*C*T*A C* C G A A A A G G*C G G*C*G -5'

ON 5503 inverted repeat

5'- G*C*T*A C* C G A A A A G G*C G G*C*G -3'

ON 5506 antisense

3'- G*C*T*A C* C G A A A A G G*C G G*C*G - C16 -5'

ON 5505 inverted repeat

5'-G*C*T*A C* C G A A A A G G*C G G*C*G -C16- 3'

ON 5959 antisense

3' G*C*G G A A G*T*T G G A T*C*T G*C 5'

For comparative animal studies a mouse specific antisense oligonucleotide was synthesized:

ON 5468 mouse $\alpha_V$ antisense

3' G*C*T A C*C G A C*G A G G G C*C*C*G 5'

* is a phosphorothioate residue, and C is Propinyl-dC, T is Propinyl-dU

Example 2: Uptake of the ON 5543 antisense ON and $\alpha_v$ protein reduction.

Uptake studies were first performed to examine whether the ON 5543 $a_v$ antisense linked to FITC was taken up into osteoclasts. *Ex vivo* rabbit osteoclasts, plated on glass (Fig.2) or dentine (not shown), internalized the fluorescent ON, with the highest levels observed in numerous intracellular vacuoles (Fig. 2); nuclei generally showed low uptake.

To examine whether the antisense ON reduced $a_v$ integrin synthesis, large numbers of rabbit osteoclasts were generated *in vitro* from the adherent bone marrow cell fraction cultured for 10 days in the presence of 10 nM 1,25-dihydroxy vitamin $D_3$. TRAP-positive multinucleated osteoclasts fully active in resorbing bone and retracting in response to $10^{-7}$ M salmon calcitonin appeared by the 6th day of culture and progressively increased with time. Cultures were then cleaned from contaminating stromal cells by mild trypsinization, and those populations containing >80% of cells of the osteoclast lineage (both mature osteoclasts and TRAP-positive putative mononuclear precursors) were treated with the ON 5543 (Table I) for 48 hours.

Example 3: Immunoblotting.

Cell extracts from 90% confluent cultures were prepared with TBS buffer (10 mM Tris-HCl, pH 7.4, and 0.9% NaCl) containing 0.5% Triton X-100 and protease inhibitors. Lysates were maintained at 4°C throughout the procedure. Lysates were centrifuged for 10 min at 11,000 g to remove nuclei and cell debris, TCA-precipitated and then solubilized in Laemmli buffer. 30 μg cell protein was subjected to SDS-PAGE (8% acrylamide gel for $a_v$, $b_3$ and actin and 12% for $p21^{WAF/CIP1}$, bcl-2 and bax), and proteins were transferred onto nitrocellulose filters.

Filters were then incubated with the primary antibody at 4°C overnight, washed and incubated with horseradish peroxidase conjugated secondary antibody at room temperature for 90 min, washed and detected by ECL.

Immunoblotting analysis (Fig. 3A) demonstrated that 1μM ON 5543 antisense reduced the level of $\alpha_v$ protein compared to untreated cultures and to cultures treated with the control ONs. In contrast, no significant change in the $\beta_3$ integrin subunit or actin expression was observed. Similar results were obtained using mouse osteoclasts generated *in vitro* as described by Tanaka et al. (32) and treated with mouse-specific (Table I) $\alpha_v$ antisense and control ONs (data not shown).

Example 4: Cell Adhesion Measurement.

Osteoclasts were plated on 13 mm diameter round coverslips or 4x4 mm bovine bone slices or dentine discs (6 mm diameter) from elephant ivory, and incubated in the presence of ON s for different times. At the end of incubation, non-adhering cells were removed by extensive washing in PBS (3x), and attached cells were fixed in 3% paraformaldehyde in PBS for 15 min at room temperature, rinsed in PBS (3x), stained for TRAP enzyme and observed by phase contrast microscopy. Adhesion was evaluated counting the number of TRAP-positive multinucleated osteoclasts per each coverslip or bone /dentine slice.

The ON 5543 $\alpha_v$ antisense inhibited *ex vivo* rabbit osteoclast adhesion to glass and to bone or dentine, and bone resorption. The titration curves were biphasic and suggested that activity can be seen at concentrations as low as $2x10^{-10}$ μM (p<0.05, Fig. 4). This high potency was likely to be due to a local concentrating effect on the substrate support. In fact, a FITC modified ON 5543 (FITC labeled ON 5543) was rapidly adsorbed to the dentine matrix, rising to a maximum at 4 hours (Fig. 5). Similar results were obtained for the FITC-ON 5543 mismatch ON adsorption to dentine and glass (not shown). Likewise, adsorption to glass increased with time and ON concentration (not shown). However, the relative amounts of FITC-labeled ON adsorbed were approximately 30 fold less than that observed with dentine.

Fig. 6 shows that all three control ONs were ineffective (p=ns ) at inhibiting *ex vivo* osteoclast adhesion and resorption relative to the 5543 antisense ON (p<0.0001). Comparative study showed a greater activity of the 5543 antisense ON (20 mM, adhesion 68% and resorption 65% inhibition vs. nil) relative to the AS3 (20 mM, adhesion 53% and resorption 50% inhibition vs. nil) and AS4 (20 mM, adhesion 48% and resorption 50% inhibition vs. nil, n=3-8; p<0.001) $a_v$ antisense ONs that were shown to inhibit melanoma cell adhesion in culture (37).

Example 5: Bone resorption measurement.

Bone resorption was evaluated according to Prallet et al. (33). Osteoclasts were plated onto bone or dentine slices and incubated in the continuous presence of the ONs for 24-48 hours. At the end of incubation, adherent TRAP-positive multinucleated cells were counted under light microscopy, then removed by sonication for 2 min in 0.01% NaOCl. Slices were rinsed twice in distilled water (20 min), and stained for 4 min in 1% toluidine blue in 1% sodium borate, and observed by conventional light microscopy with a 20x objective. The number of resorption pits in at least 5 bone slices or dentine discs for each point was counted in three visual categories, according to Prallet et al., (33). For each experiment, the mean control (vehicle only) value of the pit area index was used as a base to calculate each value as a percentage of the mean control value (31). Alternatively, bone resorption was enumerated as pit number per osteoclast, and normalized to control values of resorption in the presence of vehicle only.

Treatment of osteoclasts generated *in vitro* with the ON 5543 antisense, but not control, ONs showed inhibition of adhesion and resorption equivalent to that observed with *ex vivo* osteoclasts (data not shown). When this fraction was cultured during the 10 days necessary for differentiation in the continuous presence of ON 5543 antisense ON , a significant reduction not only of TRAP-positive multinucleated cells, but also of TRAP-positive putative osteoclast mononuclear precursors was observed. Again, all control ONs were ineffective (Fig. 7). These results indicate efficacy of the antisense ON 5468 also at the early stage of osteoclast development and validate the molecular analysis performed in this study using this cell population.

Example 6: Substrat specificity

The substrate-specificity of the ON 5543 (abbreviation 5543) effect on osteoclast adhesion was determined (Fig. 8). A concentration-dependent effect on adhesion to serum and vitronectin (Fig. 8A and B) was observed (with 63% inhibition at 20 $\mu$M, $p<0.001$). In the case of adhesion to fibrinogen and fibronectin (Fig. 8C and D) there were small, but statistically significant ($p<0.01$), reductions in the number of osteoclasts attached after 24 hours, whereas adhesion to fibrillar collagen was unaffected (Fig. 8E, p=ns ). The effect of ON 5543 antisense on osteoclast adhesion was compared with that of the function blocking monoclonal antibody 23C6 (to human $\alpha_v\beta_3$, but cross reactive to the rabbit receptor) (Fig. 8F). The inhibitory effect of 23C6 was most prominent on cells plated onto serum and vitronectin, with the responses to ON 5543 paralleling that of 23C6 on all matrix proteins tested.

Example 7: Determination of Apoptosis.

Cell morphology was evaluated by phase contrast microscopy. Bisbenzimide, which specifically binds to the Adenine-Thymidine regions of DNA, was used for nuclear staining. Cells were fixed in Carnoy's fixative (methanol-glacial acetic acid 3:1), incubated for 30 min in 0.5 $\mu$g/ml bisbenzimide, rinsed (2x in distilled water), mounted in glycerol-PBS 1:1 and observed by conventional epifluorescence microscopy.

To evaluate fragmented DNA the TUNEL method was used according to manufacturer's instruction. Briefly, cells were fixed in 4% buffered paraformaldehyde, washed (2 min, 4x in distilled water) and incubated in TdT buffer for 5-10 min at room temperature. Buffer was then removed, and cells were incubated in TdT- and Biotin-dUPT-containing buffer for 60 min at 37°C. Cells were then washed in TdT buffer for 15 min at room temperature, washed (2 min, 4x in distilled water), blocked in blocking reagent and incubated with avidin-conjugated FITC for 30 min at 37°C. Cells were washed in PBS (5 min, 3x), counterstained with 0.5 $\mu$g/ml propidium iodide, washed in PBS (3 min), mounted and observed by conventional epifluorescence microscopy.

Morphological analysis of ON 5543 antisense ON -treated osteoclasts demonstrated cell retraction relative to control ON s (Fig 9A-D) with nuclei often showing morphological changes consistent with nuclear damage. Therefore, DNA was decorated with bisbenzimide, a compound which specifically binds the adenine-thymidine regions. After 24 h challenge by the antisense ON , nuclei appeared altered with apoptotic bodies were evident (Fig. 9H). Control treated cultures (Fig. 9F and G) showed no alteration, since nuclei were undistiguishable from those in the untreated control (Fig. 9E). To confirm apoptosis, TUNEL staining of fragmented DNA, which reveals programmed cell death at a very early stage, was performed. It was found that in ON 5543 ON -treated cells (Fig. 9I)the number of apoptotic nuclei was increased relative to untreated cells or to cells treated with control ON s (Fig. 9J).

In order to examine the intracellular mechanism inducing apoptosis, experiments were then designed to investigate the genes that were altered to promote cell death upon inhibition of $a_v$ synthesis. Immunofluorescence detection of the $p21^{WAF1/CIP1}$ protein demonstrated an increase of expression in both the mononuclear cell fraction and the osteoclasts treated with the ON 5543 $a_v$ antisense, but not with control ON s (Fig. 10).

Furthermore, immunoblotting analysis demonstrated that the ON 5543 $a_v$ antisense induced a potent reduction of Bcl-2, whereas the control ON s were ineffective. In all the conditions tested, expression of Bax remained unchanged (Fig. 11A). Densitometric analysis demonstrated an approximately 50% reduction of bcl-2/bax ratio in 5543 $a_v$ anti-

sense ON -, but not in control ON -treated cells (Fig. 11B).

Example 8: Inhibition of cell Adhesion of Breast carcinoma MDA-MB231 cells and Determination of Apoptosis.

Antisense ON 5543 at 1 μM concentration was tested in carcinoma cells basically as described above and were found to inhibit MDA-MB231 cell adhesion to by about 40% whereas no cell adhesion decreased with the sense and mismatch control oligonucleotides. The antisense oligonucleotide was added to MBA-MB231 cells settled onto fibrinogen-coated wells at 0.01 to 1 μM concentrations. Cells were challenged for 24 hrs prior to staining with crystal violet. TUNEL staining of fragmented DNA again revealed programmed cell death of the cancer cells.

Example 9: Inhibition of cell Adhesion of GCT23 cells.

Antisense S96 5473 at 1 μM concentration was tested in carcinoma cells basically as described above and were found to inhibit GCT23 cell adhesion by about 40% whereas cell adhesion was not decreased with the sense and inverted control oligonucleotides.

Example 10: Inhibition of cell Adhesion by oligonucleotides of different chemical modification.

ON 5959, ON 5431, ON 5504, ON 5506 and ON 5959 were all tested as described in examples 3 and 4 and found to inhibit cell adhesion. ON 5506 gave 80 % inhibition of MBA-MB231 cell adhesion.

REFERENCES

Ruoslahti, E. and B. Öbrink. 1996. *Exp. Cell Res.* 227:1-11.
Humphries, M.J. 1996. *Curr. Opin. Cell Biol.* 8:632-640.
Meredith, J.E. Jr. and Schwartz, M.A. 1997. *Trends Cell Biol.* 7:146-150.
Suda, T., I. Nakamura, E. Jimi and N. Takahashi. 1997. *J. Bone Min. Res.* 12:869-879.
Väänänen, H.K. and M.A. Horton. 1995. *J. Cell Sci.* 108:2729-1732.
Väänänen, H.K. 1996. Osteoclast function: Biology and Mechanisms. In *Principles of Bone Biology.* J.P. Bilezikian, L.G. Raisz and G.A. Rodan editors, Academic Press, London. 103-113.
Ross, F.P., J. Chappel, J.I. Alvarez, D. Sander, W.T. Butler, M.C. Farach Carson, K.A. Mintz, P.G. Robey, S.L. Teitelbaum and D. Cheresh. 1993. *J. Biol. Chem.* 268:9901-9907.
Davies, J., J. Warwick, N. Totty, R. Philp, M. Helfrich and M.A. Horton. 1989. *J. Cell Biol.* 109:1817-1826.
Nesbitt, S., A. Nesbit, M. Helfrich and M.A. Horton. 1993. *J. Biol. Chem.* 268: 16737-16745.
Humphries, M.J. 1990. *J. Cell Sci.* 97:585-592.
Yamada, K.M. 1991. *J. Biol. Chem.* 266:12809-12812.
Bossy, B. and L.F. Reichardt. 1990. *Biochemistry* 29:10191-10198.
Ho, P.T.C., and D.R. Parkinson. 1997. *Semin. Oncol.* 24:187-202.
Sharma, H.W. and R. Narayanan. 1995. *Bioassay* 17:1005-1063.
Zon, G. 1995. *Tox. Letts.* 82/83:419-424.
Wagner, R.W. 1995. The state of the art in antisense research. *Nat. Med.* 1:1116-1118.
Stein, C.A. and Cheng, Y.C. 1993. *Science* 261:1004-1012.
Shakin, S.H. and S.A. Liebhaber. 1986. *J. Biol. Chem.* 261:16018-16025.
Uhlmann, E. and A. Peyman. 1990. *Chem. Rev.* 90:543-584.
Schlingensiepen, R., W. Brysch, and K.-H. Schlingensiepen. 1997. Antisense - from technology to therapy (lab manual and textbook) Blackwell Science Ex Libris Vol. 6.
Dean, N.M., R. McKay, T.P. Condon, and C.F. Bennett. 1994. *J. Biol. Chem.* 269: 16416-16424.
Bennett, C.F. *Cell Adhesion Molecules Meeting.* NHLI, London: IBC UK conferences.
Rojanasakul, Y. 1996. *Adv. Drug Delivery Rev.* 18:115-131.
Calbretta, B., T. Skorski, C. Szczylik and G. Zon. 1993. *Cancer Treat. Rev.* 19:69-179.
Peyman A. and E. Uhlmann. 1996. *Biol. Chem.* 377:67-70.
Horton, M.A., D. Lewis, K. McNutly, J.A.S. Pringle, K. Fuller, and T.J. Chambers. 1985. *Cancer Res.* 45:5663-5669.
Freed, E., J. Gailit., P. van der Geer, E. Ruoslahti and T. Hunter. 1989. *EMBO J.* 8:2955-2965.
Iyer, R.P., W. Egan, J.B. Regan, and S.L. Beucage. 1990. *J. Am. Chem. Soc.* 112:1253-1254.
Ojwand, J.O., S.D. Mustain, H.B. Marshall, T.S. Rao, N. Chaudhary, D.A. Walker,
M.E. Hogan, T. Akiyama, G.R. Revankar, A. Peyman, E. Uhlmann and R.F. Rando. 1997. *Biochemistry* 36:6033-6045.
Chambers, T.J., P.A. Revell, K. Fuller and N.A. Athanasou. 1984. *J. Cell Sci.* 66:383-389.
Caselli, G.F., M. Mantovanini, C.A. Gandolfi, M. Allegretti, S. Fiorentino, L.
Pellegrini, G. Melillo, R. Bertini, W. Sabbatini, R. Anacardio, G. Clavenna, G. Sciortino and A. Teti. 1997. *J. Bone Min. Res.* 12:972-981.
Tanaka, S., M. Amling, L. Neff, A. Peyman, E. Uhlmann, J.B. Levy and R. Baron. 1996. *Nature* 383:528-531.
Prallet, R., P. Male, L. Neff, and R. Baron. 1992. *J. Bone Min. Res.* 7:405-414.
Gavrieli, Y., Y. Sherman, S.A. Bem-Sasson. 1992. *J. Cell Biol.* 119:493-501.
Altschul, S.F., W. Gish, W. Miller, E.W. Meyers and D.J. Lipman. 1990. *J. Mol. Biol.* 215:403-410.
Suzuki, S., W.S.R. Argraves, H. Arai and T. Krusius. 1986. *Proc. Natl. Acad. Sci. USA* 83:8614-8618.
Nip, J., S.A. Rabbani, H.R. Shibata and P. Brodt. 1995. *J. Clin. Invest.* 95:2095-2103.
Horton, M.A. 1997. *Int. J. Biochem. Cell Biol.* 29:721-725.
Rodan, S.B. and Rodan, G.A. 1997. *J. Endocrinol.* 154:S47-S56.
Flanagan, W.M., L.L. Su and R.W. Wagner. 1996. *Nature Biotech.* 14:1139-1145.
Wagner, R.W. 1994. *Nature*, 372:333-335.
Khaled, Z., L. Benimetskaya, R. Zelster, T. Kahn, H.W. Sharma, R. Narayanan and
C.A. Stein. 1989. *Nuc. Acid Res.* 24:737-645.
Stein, C.A. 1995. *Nature Med.* 1:1119-1121.
Laitala, T. and H.K. Väänänen. 1994. *J. Clin. Invest.* 93:2311-2318.
Laitala-Leinonen, T., M.L. Howell, G.E. Dean and H.K. Väänänen. 1996. *Mol. Biol. Cell* 7:129-142.
Udagawa, N., J. Wada, J. Findlay, D.M. Hamilton and T.J. Martin. 1996. *Bone,* 18:511-516.
Inui, T., O. Ishibashi, T. Inaoka, Y. Origane, M. Kumegawa, T. Kokubo and T. Yamamura. 1997. *J. Biol. Chem.* 272:8109-8112.
Lakkakorpi, P.T., M.A., Horton, M.H. Helfrich, E.-K. Karhukorpi and H.K. Väänänen. 1991. *J. Cell Biol.* 115:1179-1186.
Hale, A.J., Smith, C.A., Sutherland, L.C., Stoneman, V.E.A., Longthorne, V.L.,
Culhane, A.C. and Williams, G.T. 1996. *Eur. J. Biochem.* 236:1-26.
White, E. 1996. *Genes Develop.* 10:1-15.
Brooks, P.C., R.A. Clark and D.A. Cheresh. 1994. *Science* 264:569-571.
Brooks, P.C., A.M.P. Montgomery, M. Rosenfield, R.A. Reisfield, T. Hu, G. Klier and D.A. Cheresh. 1994. *Cell* 79:1157-1164.
Harper, J.W., Adami, G.R., Wei, N., Keyomarsi, K. and Elledge, S.J. 1993. *Cell* 75:805-816.
Strömblad, S., Becker, J.C., Yebra, M., Brooks, P.C. and Cheresh, D.A. 1996. *J. Clin. Invest.* 98:426-433.
Deng, C., Zhang, P., Harper, J.W., Elledge, S.J. and Leder. P. 1995. *Cell* 82:675-684.
McDonald, E.R. III, Wu, G.S., Waldman, T. and El-Deiry, W.S. 1996. *Cancer Res.* 56:2250-2255.
Boudreau, N., Werb, Z. and Bissell, M.J. 1996. *Proc. Natl. Acad. Sci. USA.* 93:3509-3513.
Wu, R.-C. and Schöntal, A.H. 1997. *J. Biol. Chem.* 271:29091-29098.

FIGURE LEGENDS

Fig. 1 - The ON 5543 ("5543") $\alpha_V$ sites and sequence and the rabbit $\alpha_V$ start site sequence.

(A) The human $\alpha_V$ nucleotide site sequence is shown with the ON 5543 $\alpha_V$ included below the sequence. All sequence information is illustrated in their 5' to 3' direction with the sequence positions labeled as shown in the EMBL database. The black arrows represent the oligonucleotide primer site used in the RT-PCR cloning of the rabbit $\alpha_V$ translational start site.

(B) Control PCR reaction showing the products when using the $\alpha_V$ primers.

(C) RT-PCR of the rabbit $\alpha_V$ start site from a spleen and bone marrow total RNA pool, as compared to a positive control human $\alpha_V$ cDNA. Each sample was reamplified using nested primers (hence the double products in each line).

(D) The rabbit start sequence is shown with the 5543 $\alpha_V$ ON sequence capitalized and double underlined. One base is different between the rabbit and human $\alpha_V$ cDNAs, with a T replacing a C at position 83 (due to primer degeneracy with a Y at this position).

Fig. 2 - FITC-5543 $\alpha_v$ uptake in rabbit osteoclasts.
*Ex vivo* isolated rabbit osteoclasts were settled onto serum-coated glass and incubated with FITC labeled $\alpha_v$ 5543 antisense ON at 1 $\mu$M for 24 hours.

(A) Top view of a multinucleated osteoclast showing distribution of FITC-5543 antisense ON to granular structures within the cytoplasm; note that nuclei are negative.
(B) Side view of osteoclasts, taken through plane of section indicated by arrow in (A), showing granular distribution of antisense ON and absence in nuclei (glass upon which osteoclast is adherent is shown by the dotted line). Similar results were observed with osteoclasts on dentine (not shown). Magnification: X 2000.

Fig. 3 - The $\alpha_v$ antisense ON s reduce $\alpha_v$ protein expression.
Rabbit bone marrow adherent cell fraction was cultured for 10 days with 10 nM 1,25-dihydroxy vitamin $D_3$ in order to differentiate a large number of TRAP-positive osteoclasts. Cultures were then treated with 1 $\mu$M $\alpha_v$ antisense and control ON s for 24 hours, lysed, subjected to SDS-PAGE (30 $\mu$g cell protein) and immunoblotted with antibodies recognizing $\alpha_v$ and $\beta_3$ integrin subunits, and actin. Primary antibodies were diluted 1:250. Secondary antibodies were diluted 1:5000. $a_v$ protein level from the above gel was quantified by densitometric analysis, normalized versus actin, and expressed in arbitrary units as percent with respect to control.

Fig. 4 - The 5543 $\alpha_v$ antisense ON inhibits osteoclast adhesion and resorption.
The $\alpha_v$ ON was added to rabbit osteoclasts settled onto serum-coated glass (A) or dentine chips (B) at increasing concentrations ($5x10^{-13}$ to 100 $\mu$M). Cells were challenged for 24 hours prior to TRAP and/or pit staining. n= 8; ±S.E.M. ○ adhesion to serum-coated glass. ● adhesion to dentine chips. □ resorption. △ nil. *, $p$<0.05; **, $p$<0.01; ***, $p$<0.001; **** , $p$<0.0005.

Fig. 5 - The 5543 $\alpha_v$ antisense ON binding to dentine.

(A) FITC-linked $\alpha_v$ antisense ON binding to dentine at increasing concentrations was assessed with time from 30 minutes to 24 hours.
(B) Represents the residual fluorescence within the media at the start (0 hours) and end point (24 hours). n=6; ±S.E.M. raw, arbitrary fluorescence units plotted versus time. ■ nil. □ 0.2 mM. ● 2 mM. ○ 20 mM.

Fig. 6 - Comparison of 5543 $\alpha_v$ antisense and control ONs on rabbit osteoclast function.
Studies were performed using the $\alpha_v$ antisense ON and three relevant specificity controls. Antisense, mismatch, inverted, and sense ONs were incubated with *ex vivo* osteoclasts (0.2 to 20 $\mu$M) for 24 hours.

(A) Adhesion to serum-coated glass.

(B) Adhesion to dentine discs. (C) Resorption. ● antisense. ○ mismatch. ■ inverted. □ sense. △ nil n=3; ±S.E.M. *, $p$<0.001.

Fig. 7 - The 5543 $\alpha_v$ antisense ON effect on osteoclasts differentiated *in vitro*.
The rabbit bone marrow adherent cell fraction was cultured for 10 days with 10 nM 1,25-dihydroxy vitamin $D_3$ and 1 mM of the indicated ONs. At the end of incubation, cells were stained for TRAP enzyme and the number of TRAP-positive (A) mononuclear (MNC) and (B) multinucleated (PNC) cells were counted and expressed as a percent rate versus untreated cultures. a'sense, antisense ON. sense, sense ON. mism, mismatch ON . n=3; ±S.E.M. ***, $p<10^{-6}$

Fig. 8 - Dose response effects of the 5543 $\alpha_v$ antisense ON on *ex vivo* rabbit osteoclasts adhered to different extracellular matrix proteins.
Parallel studies were performed with the sense control ON (here shown at 20$\mu$M concentration only). Cultures were treated for 24 hours post attachment onto the indicated substrate protein-coated glass. (A) FBS, Fetal Bovine Serum. (B) Vn, Vitronectin. (C) Fb, Fibrinogen. (D) Fn, Fibronectin. (E) Fibrillar Collagen. (F) Table showing comparison between the effect of the $\alpha_v\beta_3$ neutralizing antibody 23C6 and the 5543 $\alpha_v$ ON. In parenthesis the percent variation vs. nil is shown. a/s, antisense ON (from left 0 $\mu$M, 0.2 $\mu$M, 2 $\mu$M, 20 $\mu$M). s, sense ON (20 $\mu$M). n=3; ±S.E.M. *, $p$<0.05; ** , $p$<0.01; ***, $p$<0.001.

Fig. 9 - The 5543 $\alpha_v$ antisense ON effect on osteoclast morphology and apoptosis.
*Ex vivo* rabbit osteoclasts were incubated with the indicated ONs for 24 hours. (A-D) Phase contrast micrographs.

Arrow indicates a retracted osteoclast. Magnification X 1400.
(E-H) Bisbenzimide nuclear staining. Arrow indicates apoptotic bodies. Magnification X 1400. (I-L) TUNEL staining of fragmented DNA. Magnification X 700. A,E,I: control (c). B,F,J: sense ON (s). C,G,K: mismatch ON (m). D,H,L: antisense ON (a/s).

Fig. 10 - The 5543 $a_v$ antisense ON stimulates $p21^{WAF1/CIP1}$ immunoreacttivity.
Untreated *ex vivo* rabbit osteoclasts (A) and osteoclasts incubated for 48 hours with 1 mM of (B) sense, (C) mismatch and (D, E) antisense ONs, were fixed and subjected to immunofluorescence staining with an antibody recognizing the $p21^{WAF1/CIP1}$ protein. Arrows indicate $p21^{WAF1/CIP1}$-positive osteoclasts. Magnification X 1000. (F) The number of $p21^{WAF1/CIP1}$ immunoreactive mononuclear cells and the total number of mononuclear cells present in at least 5 random microscopic field (objective 20X) were counted in control and in antisense (a'sense)-, sense- and mismatch (mism)-treated cultures, and the percent of positive versus total cells calculated. n=3±S.E.M. ***p<0.0001.

Fig. 11 - Expression of the bcl-2 and bax genes.
Cells of the osteoclast lineage were obtained as described in Fig. 3 and treated with 1mM $a_v$ antisense and control ONs for 48 hours. Cells were then lysated and subjected to analysis of bcl-2 and bax protein levels by Western blotting as described in Methods. The bcl-2 and bax protein levels were then quantified by densitometry upon normalization versus actin expression. The bars represent the relative ratio as measured by densitometric analysis and not the absolute molar ratio. c: control. s: sense ON. m: mismatch ON. a/s: antisense ON.

SEQUENCE LISTING

(1) GENERAL INFORMATION:

(i) APPLICANT:
(A) NAME: Hoechst Marion Roussel Deutschland GmbH
(B) STREET: -
(C) CITY: Frankfurt
(D) STATE: -
(E) COUNTRY: Germany
(F) POSTAL CODE (ZIP): 65926
(G) TELEPHONE: 069-305-7072
(H) TELEFAX: 069-35-7175
(I) TELEX: -

(ii) TITLE OF INVENTION: Antisense Oligonucleotides for the Inhibition of Integrin av-Subunit Expression

(iii) NUMBER OF SEQUENCES: 12

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 540 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: exon
(B) LOCATION: 1..540

(xi) SEQUENCE DESCRIPTION: SEQ ID NO: 1:

```
GGCTACCGCT CCCGGCTTGG CGTCCCGCGC GCACTTCGGC GATGGCTTTT CCGCCGCGGC     60

GACGGCTGCG CCTCGGTCCC CGCGGCCTCC CGCTTCTTCT CTCGGGACTC CTGCTACCTC    120

TGTGCCGCGC CTTCAACCTA GACGTGGACA GTCCTGCCGA GTACTCTGGC CCCGAGGGAA    180

GTTACTTCGG CTTCGCCGTG GATTTCTTCG TGCCCAGCGC GTCTTCCCGG ATGTTTCTTC    240

GGCTACCGCT CCCGGCTTGG CGTCCCGCGC GCACTTCGGC GATGGCTTTT CCGCCGCGGC    300
```

CCGATGGCGA GGGCCGAACC GCAGGGCGCG CGTGAAGCCG CTACCGAAAA GGCGGCGCCG 360

CCGATGGCGA GGGCCGAACC GCAGGGCGCG CGTGAAGCCG CTACCGAAAA GGCGGCGCCG 420

CCGATGGCGA GGGCCGAACC GCAGGGCGCG CGTGAAGCCG CTACCGAAAA GGCGGCGCCG 480

CCGATGGCGA GGGCCGAACC GCAGGGCGCG CGTGAAGCCG CTACCGAAAA GGCGGCGCCG 540

(2) INFORMATION FOR SEQ ID NO: 2:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..24

(xi) SEQUENCE DESCRIPTION: SEQ ID NO: 2:

CGGCGATGGC TTTTCCGCCG CGGC 24

(2) INFORMATION FOR SEQ ID NO: 3:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..26

(xi) SEQUENCE DESCRIPTION: SEQ ID NO: 3:

GTGCCGCGCC TTCAACCTAG ACGTGG 26

(2) INFORMATION FOR SEQ ID NO: 4:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..20

(xi) SEQUENCE DESCRIPTION: SEQ ID NO: 4:

GAAGCCGCTA CCGAAAAGGC 20

(2) INFORMATION FOR SEQ ID NO: 5:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..18

(xi) SEQUENCE DESCRIPTION: SEQ ID NO: 5:

CGCGTGAAGC CGCTACCG 18

(2) INFORMATION FOR SEQ ID NO: 6:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..18

(xi) SEQUENCE DESCRIPTION: SEQ ID NO: 6:

GCTACCGAAA AGGCGGCG 18

(2) INFORMATION FOR SEQ ID NO: 7:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..18

(xi) SEQUENCE DESCRIPTION: SEQ ID NO: 7:

GCTGCCGAGA GAGCAACG 18

(2) INFORMATION FOR SEQ ID NO: 8:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..17

(xi) SEQUENCE DESCRIPTION: SEQ ID NO: 8:

GCGGAAGTTG GATCTGC 17

(2) INFORMATION FOR SEQ ID NO: 9:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..18

(xi) SEQUENCE DESCRIPTION: SEQ ID NO: 9:

GCTACCGACG AGGGCCCG 18

(2) INFORMATION FOR SEQ ID NO: 10:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..18

(xi) SEQUENCE DESCRIPTION: SEQ ID NO: 10:

GCTACCGAAA AGGCGGCG 18

(2) INFORMATION FOR SEQ ID NO: 11:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..18

(xi) SEQUENCE DESCRIPTION: SEQ ID NO: 11:

CGATGGCTTT TCCGCCGC 18

(2) INFORMATION FOR SEQ ID NO: 12:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..17

(xi) SEQUENCE DESCRIPTION: SEQ ID NO: 12:

GCGGAAGTTG GACCTGC 17

Table 1: Nucleotide sequence of SEQ ID NO. 1 and deduced amino acid sequence.

```
GGCTACCGCT CCCGGCTTGG CGTCCCGCGC GCACTTCGGC GATGGCTTTT CCGCCGCGGC
CCGATGGCGA GGGCCGAACC GCAGGGCGCG CGTGAAGCCG CTACCGAAAA GGCGGCGCCG

1 ---------+---------+---------+---------+---------+---------+ 60
LeuProLeu ProAlaTrp ArgProAla ArgThrSerAla MetAlaPhe ProProArg Arg


GACGGCTGCG CCTCGGTCCC CGCGGCCTCC CGCTTCTTCT CTCGGGACTC CTGCTACCTC
CTGCCGACGC GGAGCCAGGG GCGCCGGAGG GCGAAGAAGA GAGCCCTGAG GACGATGGAG
61---------+---------+---------+---------+---------+---------+ 120
ArgLeuArg LeuGlyPro ArgGlyLeu ProLeuLeuLeu SerGlyLeu LeuLeuPro Leu


TGTGCCGCGC CTTCAACCTA GACGTGGACA GTCCTGCCGA GTACTCTGGC CCCGAGGGAA
ACACGGCGCG GAAGTTGGAT CTGCACCTGT CAGGACGGCT CATGAGACCG GGGCTCCCTT
121---------+---------+---------+---------+---------+---------+180
CysArgAla PheAsnLeu AspValAsp SerProAlaGlu TyrSerGly ProGluGly Ser


GTTACTTCGG CTTCGCCGTG GATTTCTTCG TGCCCAGCGC GTCTTCCCGG ATGTTTCTTC
CAATGAAGCC GAAGCGGCAC CTAAAGAAGC ACGGGTCGCG CAGAAGGGCC TACAAAGAAG
181---------+---------+---------+---------+---------+---------+240
```

Table 2: Part of SEQ ID NO. 1 and localisation of the sequence-parts to which the oligonucleotides correspond:

```
GGCTACCGCT CCCGGCTTGG CGTCCCGCGC GCACTTCGGC GATGGCTTTT CCGCCGCGGC
CCGATGGCGA GGGCCGAACC GCAGGGCGCG CGTGAAGCCG CTACCGAAAA GGCGGCGCCG
1 ---------+---------+---------+---------+---------+---------+ 60

Position of ON 5543
CCGATGGCGA GGGCCGAACC GCAGGGCGCG CGTGAAGCCG CTACCGAAAA GGCGGCGCCG

Position of 5541
CCGATGGCGA GGGCCGAACC GCAGGGCGCGC GTGAAGCCG CTACCGAAAA GGCGGCGCCG

Position of AS-3
CCGATGGCGA GGGCCGAACC GCAGGGCGCGC GTGAAGCCG CTACCGAAAA GGCGGCGCCG

Position of ON 5542
CCGATGGCGA GGGCCGAACC GCAGGGCGCGC GTGAAGCCG CTACCGAAAA GGCGGCGCCG
```

Tabelle 4

| | |
|---|---|
| 5543 hum. αV antisense | GCGGCGGAAAAGCCATCG |
| 5543 hum. αV sense | CGATGGCTTTTCCGCCGC |
| 5543 hum. αV mismatch | GCAACGAGAGAGCCGTCG |
| 5543 hum. αV inverted | GCTACCGAAAAGGCGGCG |
| 5468 mouse αV antisense | GCccgGGAgcAGCCATCG |
| 5468 mouse αV sense | GCTACCGACGAGGGCCCG |
| 5468 mouse αV inverted | CGATGGCTGCTCCCGGGC |

**Claims**

1. An oligonucleotide or a derivative thereof which

   a) has a sequence that corresponds to a part of a nucleic acid which encodes an integrin $\alpha_V$ subunit and
   b) has the ability to induce apoptosis.

2. An oligonucleotide as claimed in claim 1, wherein the part of the nucleic acid to which the oligonucleotide corresponds has a length of 8 to 26 nucleotides.

3. An oligonucleotide as claimed in one or more of claims 1 and 2, which when brought into contact with a cell modulates the expression of at least one protein which is involved in apoptosis.

4. An oligonucleotide as claimed in claim 3, which induces an increase in p21 expression.

5. An oligonucleotide as claimed in one or more of claims 3 and 4, which induces a reduction in bcl-2 expression.

6. An oligonucleotide as claimed in one or more of claims 3 to 5, which causes a reduction of the bcl-2/bax ratio.

7. An oligonucleotide as claimed in one or more of claims 1 to 6, which when brought into contact with a cell induces an inhibition of cell adhesion.

8. An oligonucleotide as claimed in one or more of claims 1 to 7, which when brought into contact with an osteoclast cell induces an inhibition of bone resorption.

**9.** An oligonucleotide as claimed in one or more of claims 1 to 8, which has a sequence that corresponds to a part of the human integrin $\alpha_V$ subunit cDNA.

**10.** An oligonucleotide as claimed in one or more of claims 1 to 9, which has a sequence that corresponds to position 37-60 or position 122-147 of SEQ ID NO. 1 or a part thereof.

**11.** An oligonucleotide as claimed in one or more of claims 1 to 10, which has one of the sequences

| | |
|---|---|
| SEQ ID NO. 4: | 3'- GAAGCCGCTACCGAAAAGGC -5′ |
| SEQ ID NO. 5: | 3'- CGCGTGAAGCCGCTACCG -5′ |
| SEQ ID NO. 6: | 3'- GCTACCGAAAAGGCGGCG -5′ |
| SEQ ID NO. 7: | 3'- GCTGCCGAGAGAGCAACG -5' |
| SEQ ID NO. 8: | 3'- GCGGAAGTTGGATCTGC -5' and |
| SEQ ID NO. 12: | 3'- GCGGAAGTTGGACCTGC -5′. |

**12.** An oligonucleotide as claimed in one or more of claims 1 to 11, which has one or more chemical modifications.

**13.** An oligonucleotide as claimed in one or more of claims 1 to 12, wherein 1-5 nucleotides at the 3′ and/or the 5′ end of the oligonucleotide are modified.

**14.** An oligonucleotide as claimed in one or more of claims 1 to 13, wherein at least one non-terminal pyrimidine nucleoside and/or a phosphodiester bridge located at the 3′ and/or the 5′ end of that pyrimidine nucleoside is modified.

**15.** An oligonucleotide as claimed in one or more of claims 12 to 14, wherein each modification is independently selected from

a) the replacement of a phosphoric diester bridge located at the 3'- and/or the 5′ - end of a nucleoside by a modified phosphoric diester bridge,
b) the replacement of phosphoric diester bridge located at the 3′ - and/or the 5′ -end of a nucleoside by a dephospho bridge,
c) the replacement of a sugar phosphate molecule from the sugarphosphate backbone by an other molecule,
d) the replacement of a β-D-2′-deoxyribose by a modified sugar radical,
e) the replacement of a natural nucleoside base by a modified base,
f) the conjugation of the oligonucleotide to a molecule which influences the properties of the oligonucleotide and
g) the introduction of a 3′-3′ and/or a 5′-5′ inversion at the 3′ and/or the 5′ end of the oligonucleotide.

**16.** A process for the preparation of an oligonucleotide as claimed in one or more of claims 1 to 15, which comprises its chemical synthesis.

**17.** The use of an oligonucleotide as claimed in one or more of claims 1 to 15, as antisense oligonucleotide, as triple-helix forming oligonucleotide, as adaptamer or as ribozyme.

**18.** The use of an oligonucleotide as claimed in one or more of claims 1 to 15, for inhibiting the expression of integrin $\alpha_V$ subunit.

**19.** The use of an oligonucleotide as claimed in one or more of claims 1 to 15, for modulating the expression of at least one protein which is involved in apoptosis.

**20.** The use of an oligonucleotide as claimed in one or more of claims 1 to 15, for inducing apoptosis in a cell.

**21.** The use of an oligonucleotide as claimed in one or more of claims 1 to 15, for inhibiting cell adhesion.

**22.** The use of an oligonucleotide as claimed in one or more of claims 1 to 15, for inhibiting bone resorption.

**23.** A methode of inhibiting the expression of integrin $\alpha_V$ subunit and/or modulating the expression of a protein which is involved in apoptosis and/or inducing apoptosis and/or inhibiting cell adhesion and/or inhibiting bone resporption, wherein an oligonucleotide as claimed in one or more of claims 1 to 15 is brought into contact with a cell.

**24.** The use of an oligonucleotide as claimed in one or more of claims 1 to 15 for the preparation of a pharmaceutical composition for treating deseases which are associated with the expression or an increased expression of the integrin $\alpha_V$ subunit.

**25.** A methode for the preparation of a pharmaceutical composition, which comprises mixing an oligonucleotide as claimed in one or more of claims 1 to 15 with physiologically acceptable exipient and if appropiate suitable additives and/or auxiliaries.

**26.** A pharmaceutical composition which comprises at least one oligonucleotide as claimed in one or more of claims 1 to 15 and a physiologically acceptable exipient and if appropiate suitable additives and/or auxiliaries.

**27.** A pharmaceutical composition as claimed in claim 26, for the treatment of diseases which are assoziated with abnormal cell proliferation, cell migration, cell differentiation, angiogenesis, retinal neurite outgrowth, bone resorption, phagocytosis, immune response, signal transduction and the metastasis of neoplastic cells.

**28.** A pharmaceutical composition as claimed in claim 26, for the treatment and prevention of cancer and metastasis of cancer, the treatment and prevention of osteoporosis, the treatment of ocular diseases, chronic inflammation, psorasis, restenosis and the support of wound healing.

Fig. 1 A

**A**

```
                 αV1
    ────────────────────────►            +1  Coding Region
    CCCGGCTTGGCGTCCCGCGCACTTCGGCGATGGCTTTTCCGCCGCGGC
190 ---------+---------+---------+---------+---------+ 239
                                CGATGGCTTTTCCGCCGC 5543

                          αV2
                   ◄──────────────
    GACGGCTGCGCCTCGGTCCCCGCGGCCTCCCGCTTCTTCTCTCGGGACTC
240 ---------+---------+---------+---------+---------- 289
                   ──────────────►
                         αV2.1

    CTGCTACCTCTGTGCCGCGCCTTCAACCTAGACGTGGACAGTCCTGCCGA
290 ---------+---------+---------+---------+---------+ 339

    GTACTCTGGCCCCGAGGGAAGTTACTTCGGCTTCGCCGTGGATTTCTTCG
340 ---------+---------+---------+---------+---------+ 389
                                   ◄──────────────
                                          αV3

    TGCCCAGCGCGTCTTCCCGG
390 ---------+---------+ 409
```

Fig. 1 B - C

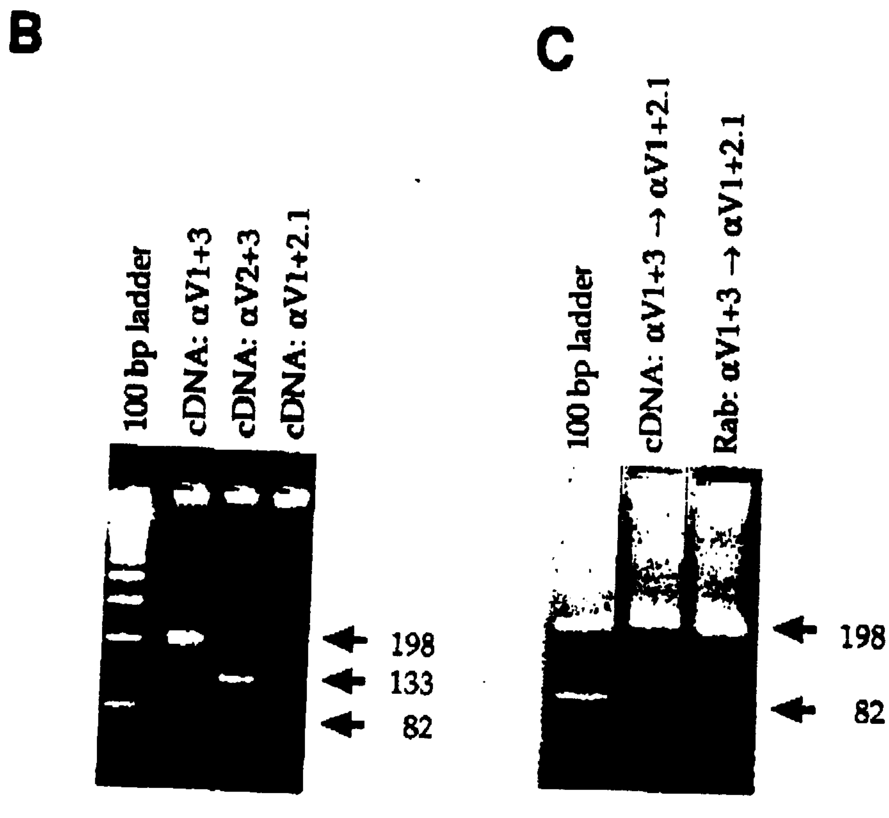
B
C
100 bp ladder
cDNA: αV1+3
cDNA: αV2+3
cDNA: αV1+2.1
198
133
82
100 bp ladder
cDNA: αV1+3 → αV1+2.1
Rab: αV1+3 → αV1+2.1
198
82

Fig. 1 D

**D**

```
                            10         20          40
                  ggcgtcccgcgcgcacttcggCGATGGCTTTTC  rab αV
                  |||||||||||||||||||||||||||||||||
ggctaccgctcccggcttggcgtcccgcgcgcacttcggcgatggcttttc  hum αV
        190       200       210       220       230

      50        60        70        80
CGCCGCggcgacggctgcgcctcggtccccgcggcctcctgct
|||||||||||||||||||||||||||||||||||||||| |||
cgccgcggcgacggctgcgcctcggtccccgcggcctcccgcttcttctc
       240       250       260       270       280
```

Fig. 2

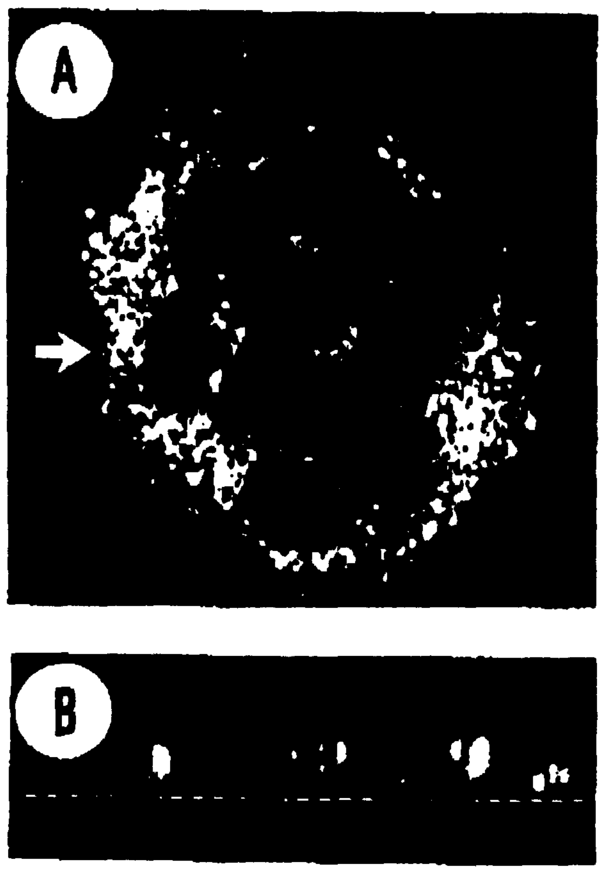

Fig. 3

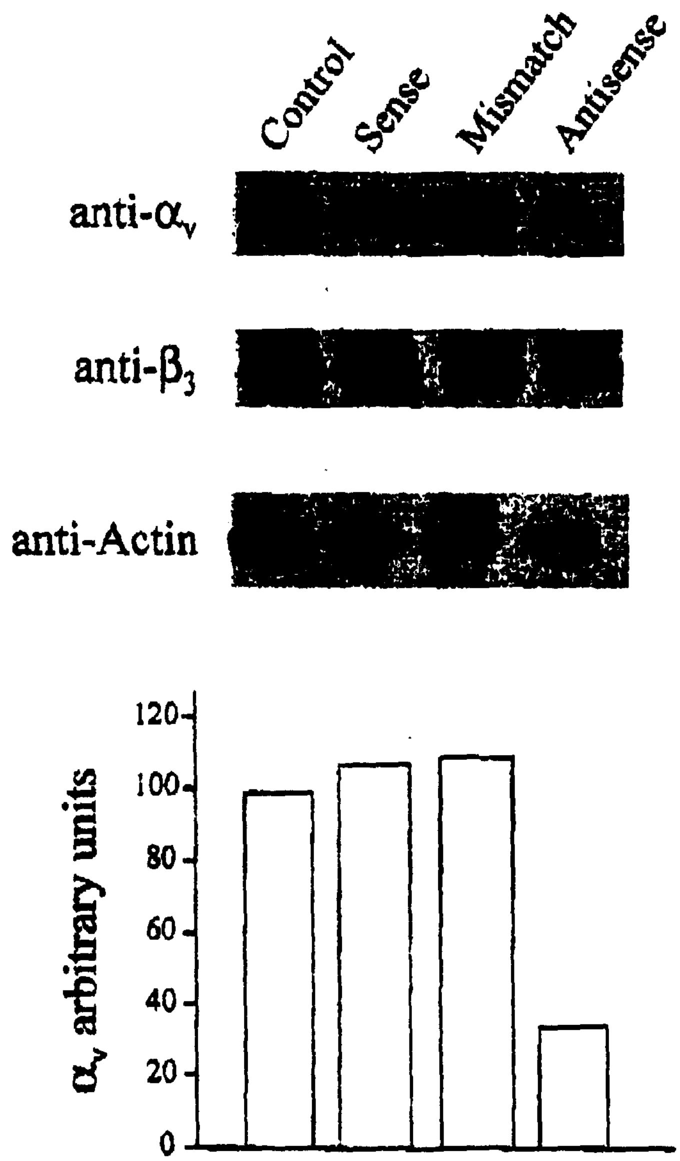
Control
Sense
Mismatch
Antisense
anti-$\alpha_v$
anti-$\beta_3$
anti-Actin
120
100
80
60
40
20
0
$\alpha_v$ arbitrary units

Fig 4

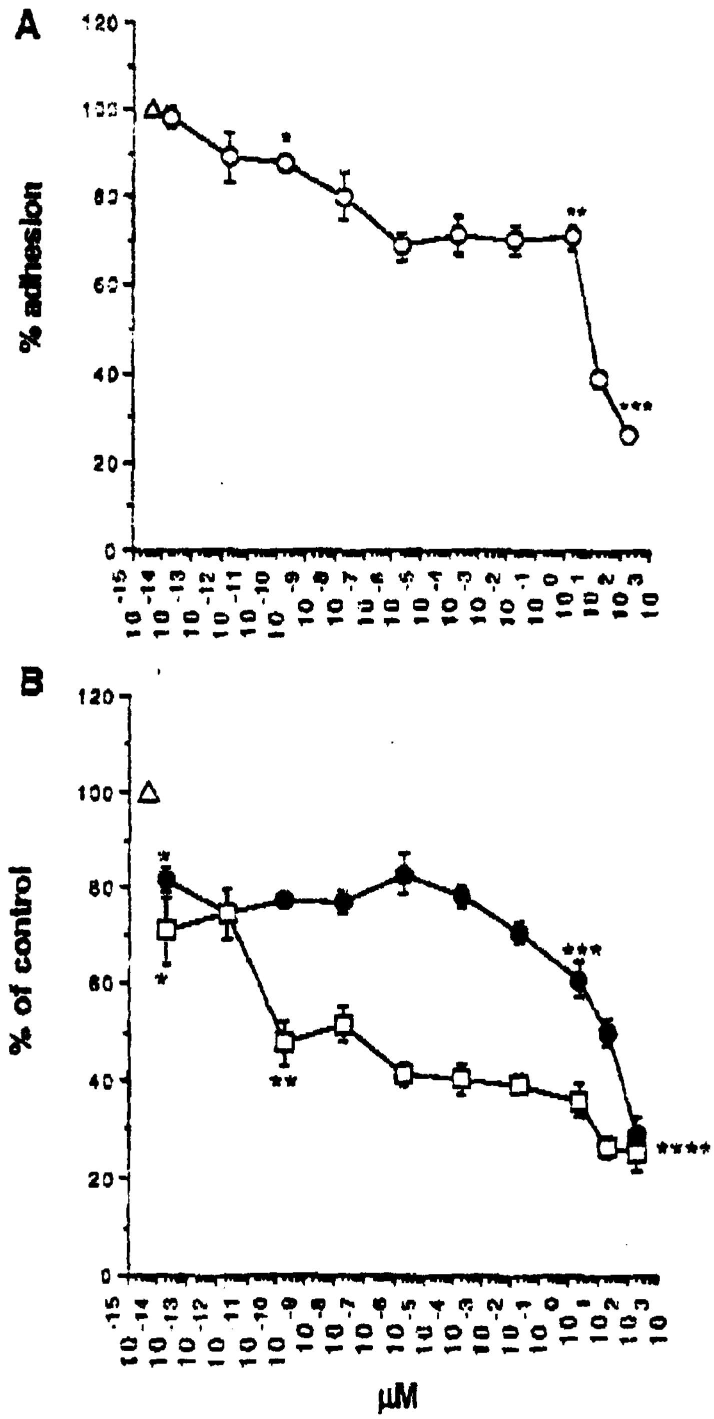

Fig. 5

A

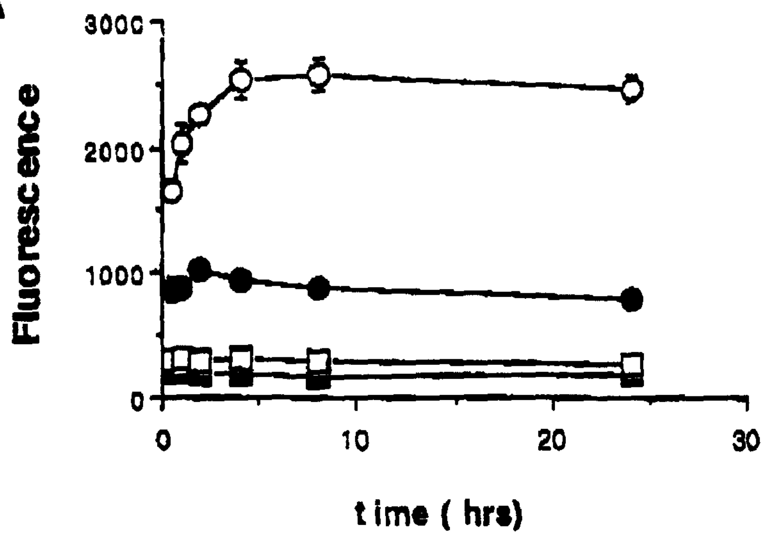


B

| | Bone | |
|---|---|---|
| [ODN] μM | t=0 | t=24 |
| nil | 36.4 | 5.04 |
| 0.2 | 485.7 | 96.93 |
| 2 | 1922 | 439 |
| 20 | 4993 | 1799 |

Fig. 6

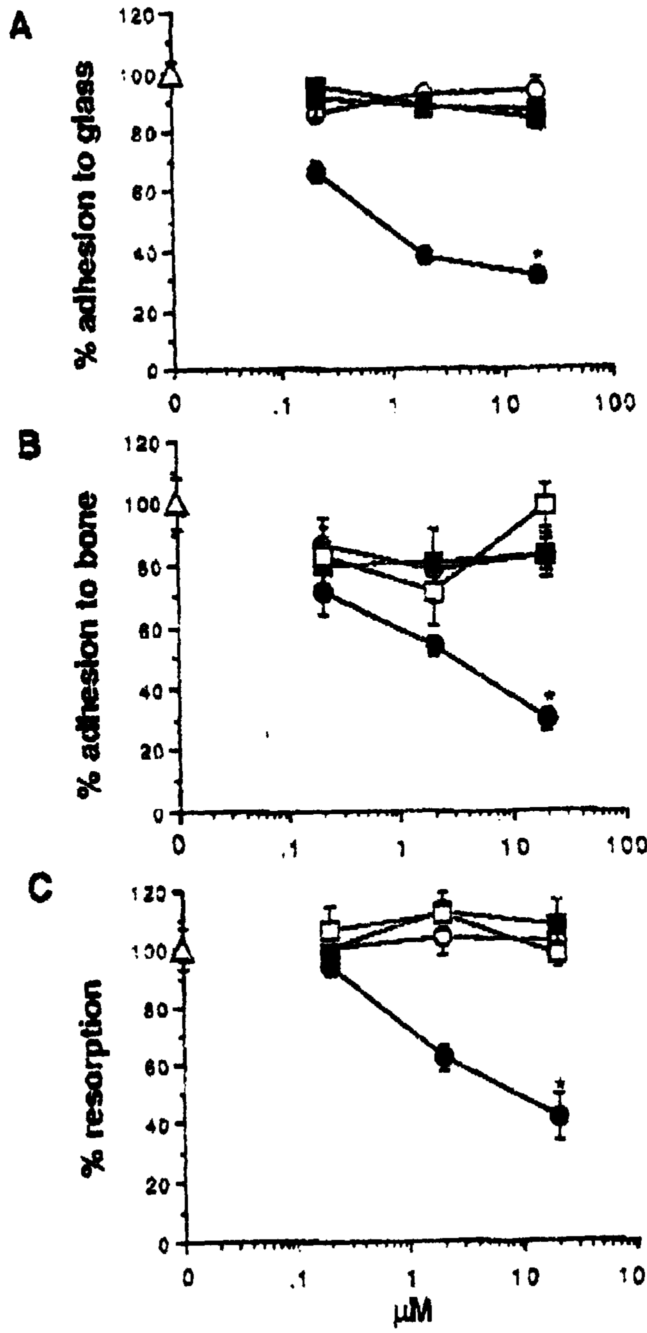
A
% adhesion to glass
120
100
80
60
40
20
0
0
.1
1
10
100
B
% adhesion to bone
120
100
80
60
40
20
0
0
.1
1
10
100
C
% resorption
120
100
80
60
40
20
0
0
.1
1
10
100
μM

Fig. 7

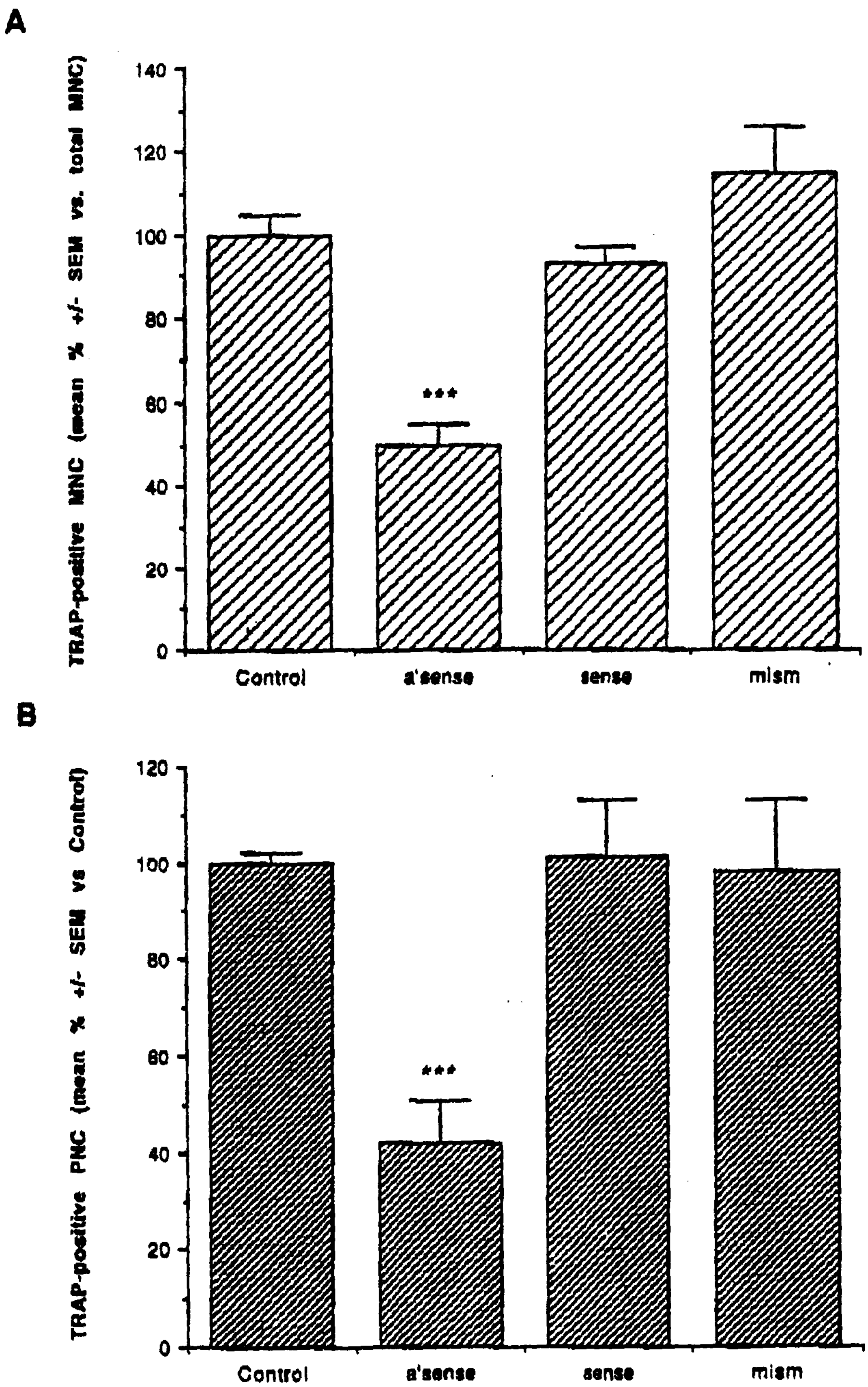
A
TRAP-positive MNC (mean % +/- SEM vs. total MNC)
140
120
100
80
60
40
20
0
***
Control
a'sense
sense
mism
B
TRAP-positive PNC (mean % +/- SEM vs Control)
120
100
80
60
40
20
0
***
Control
a'sense
sense
mism

Fig. 8

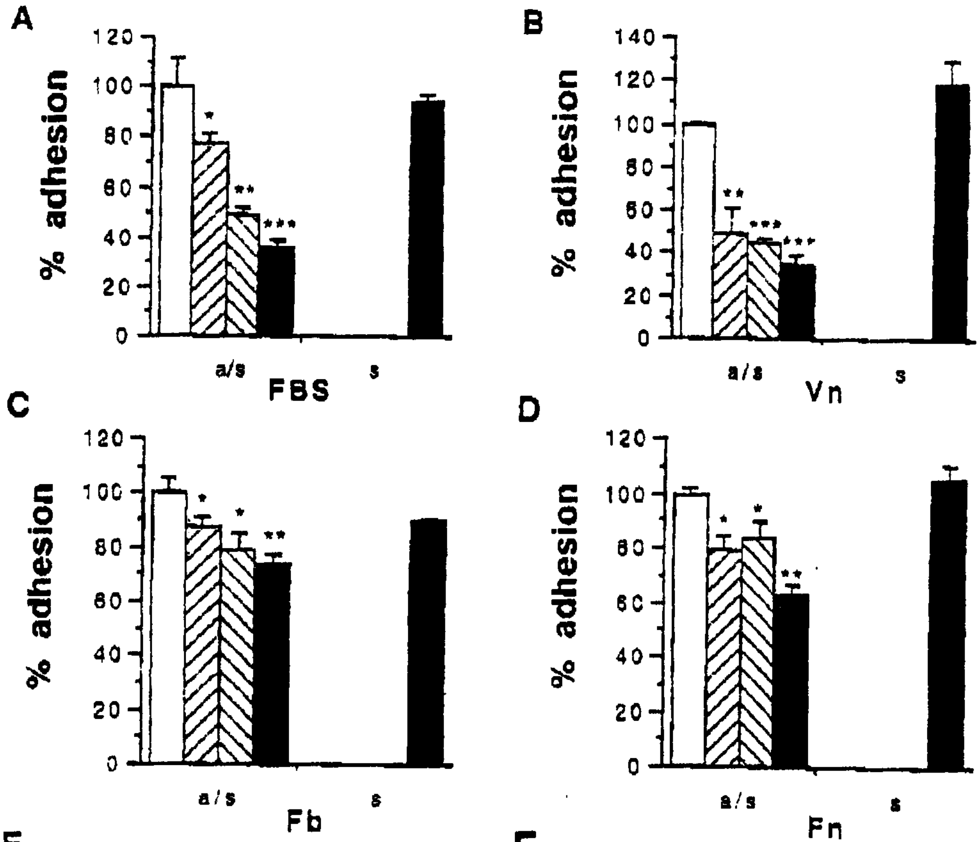


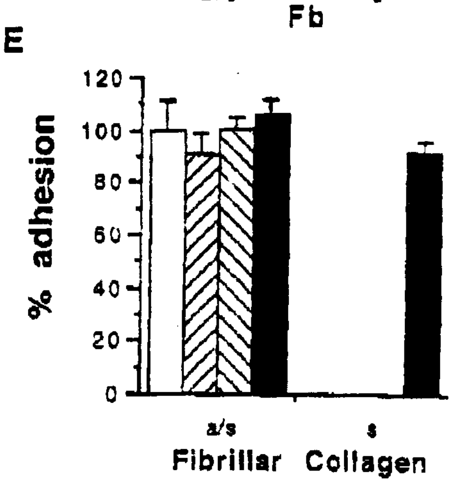


F

| | 23C6 | 5643 αV O N |
|---|---|---|
| Matrix protein | 24 hr | 24 hr |
| FBS | ↓↓↓ (50) | ↓↓↓ (60) |
| Fibrillar Collagen | →↑ (+20) | → (+5) |
| Vitronectin | ↓↓↓↓ (80) | ↓↓↓ (70) |
| Fibrinogen | ↓↓ (30) | ↓↓ (20) |
| Fibronectin | ↓↓ (35) | ↓↓ (40) |

Fig. 9

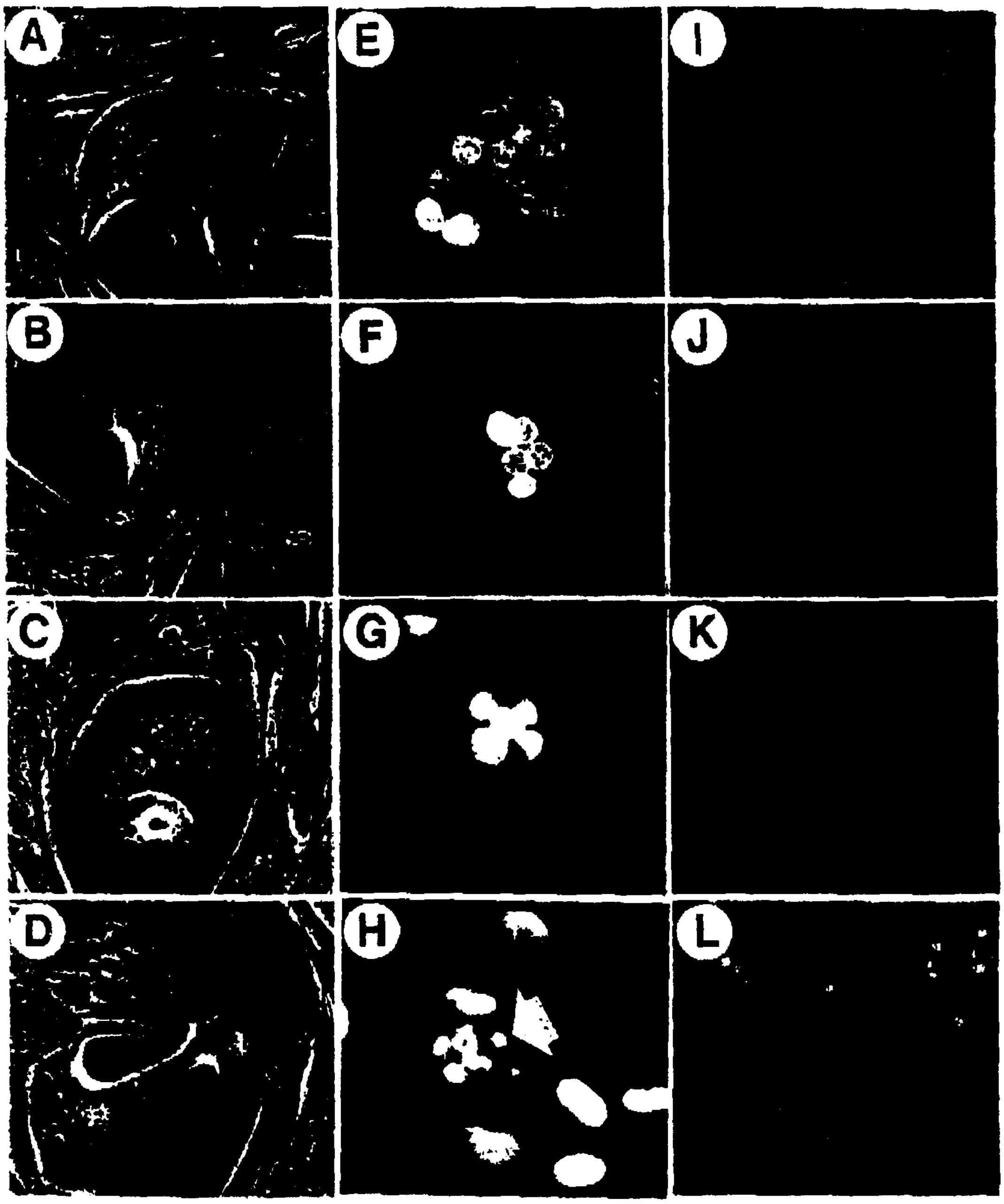

Fig. 10

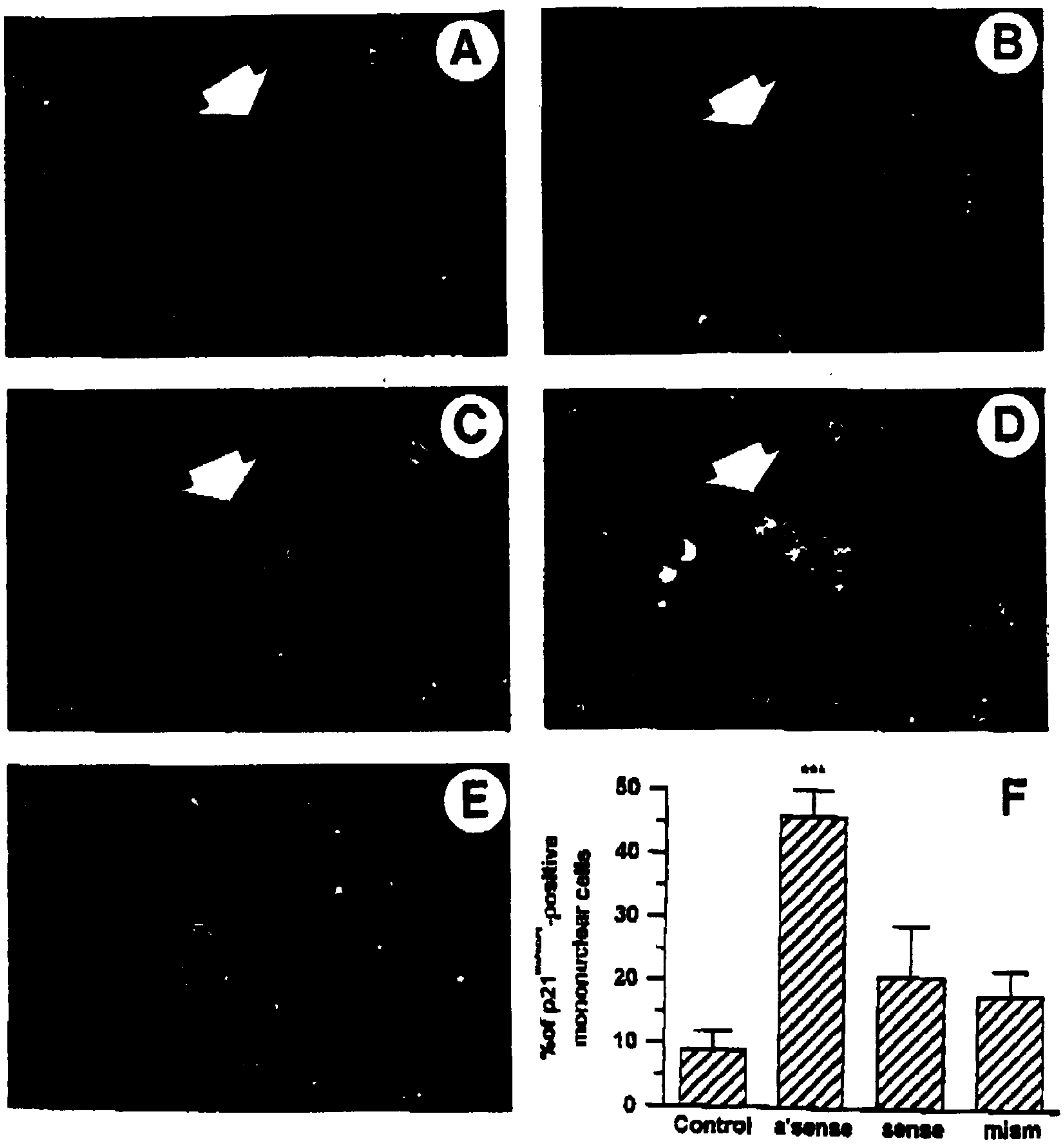
A
B
C
D
E
F
mononuclear cells
50
40
30
20
10
0
Control
a'sense
sense
mism

Fig. 11

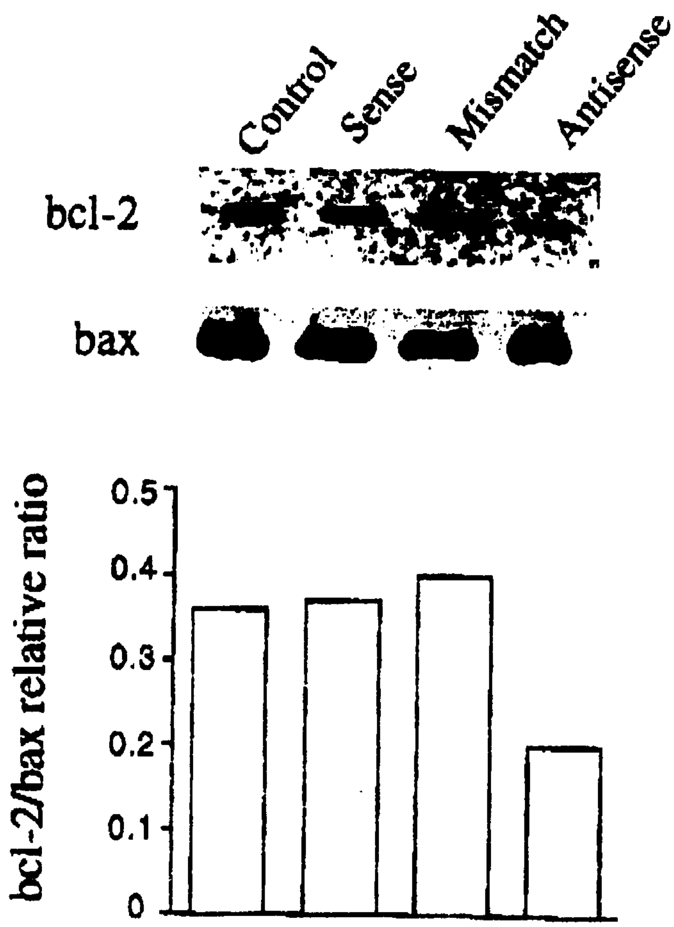
Control
Sense
Mismatch
Antisense
bcl-2
bax
bcl-2/bax relative ratio
0.5
0.4
0.3
0.2
0.1
0

European Patent Office

## PARTIAL EUROPEAN SEARCH REPORT

which under Rule 45 of the European Patent Convention shall be considered, for the purposes of subsequent proceedings, as the European search report

Application Number
EP 98 10 6989

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | TOWNSEND P ET AL: "alpha(v) integrin antisense oligodeoxynucleotides induce detachment and apoptosis of osteoclast and breast carcinoma cell." JOURNAL OF BONE AND MINERAL RESEARCH, (AUG 1997) VOL. 12, SUPP. '1!, PP. S416, ABSTRACT S252., XP002078998 | 1-9,12, 16-28 | C12N15/11 A61K31/70 C07H21/04 A61K47/48 |
| Y | * abstract * | 13-16 | |
| Y | EP 0 653 439 A (HOECHST AG) 17 May 1995 * abstract * * page 5 - page 6 * * claims * | 13-16 | |
| X,D | NIP J ET AL: "Coordinated expression of the vitronectin receptor and the urokinase-type plasminogen activator receptor in metastatic melanoma cells." JOURNAL OF CLINICAL INVESTIGATION, (1995 MAY) 95 (5) 2096-103., XP002078999 * the whole document * | 1-7, 9-12, 15-18, 21,23 | |
| | -/-- | | **TECHNICAL FIELDS SEARCHED (Int.Cl.6)** |
| | | | C12N A61K C07H |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claim 23 (as far as an in vivo method is concerned) is directed to a method of treatment of the human/animal body (Article 52(4) EPC), the search has been carried out and based on the alleged effects of the compound/composition.

| Place of search | Date of completion of the search | Examiner |
|---|---|---|
| THE HAGUE | 29 September 1998 | Andres, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 98 10 6989

| DOCUMENTS CONSIDERED TO BE RELEVANT | | | CLASSIFICATION OF THE APPLICATION (Int.Cl.6) |
|---|---|---|---|
| Category | Citation of document with indication, where appropriate, of relevant passages | Relevant to claim | |
| X | VILLANOVA J ET AL.: "ANTISENSE OLIGONUCLEOTIDES AGAINST THE ALPHA(V) INTEGRIN SUBUNIT INHIBIT OSTEOCLAST ADHESION AND BONE - RESORPTION" JOURNAL OF BONE AND MINERAL RESEARCH, (AUG 1996) VOL. 11, SUPP. 1, PP. S289, ABSTRACT M431., XP002079000 * abstract * --- | 1-9,12, 13, 15-18, 21-23 | |
| D,X | MARTIN, P. & SANES, J.: "Integrins mediate adhesion to agrin and modulate agrin signaling" DEVELOPMENT, vol. 124, October 1997, pages 3909-3917, XP002079001 * the whole document * --- | 1-7,12, 16-18, 21,23 | TECHNICAL FIELDS SEARCHED (Int.Cl.6) |
| D,X | WADA J ET AL: "CLONING OF MOUSE INTEGRIN ALPHA(V) CDNA AND ROLE OF THE ALPHA(V)-RELATED MATRIX RECEPTORS IN METANEPHRIC DEVELOPMENT" JOURNAL OF CELL BIOLOGY, (MAR 1996) VOL. 132, NO. 6, PP. 1161-1176., XP002079002 * the whole document * --- | 1-6,12, 16-18,23 | |
| D,A | BROOKS P C ET AL: "INTEGRIN ALPHAVBETA3 ANTAGONISTS PROMOTE TUMOR REGRESSION BY INDUCING APOPTOSIS OF ANGIOGENIC BLOOD VESSELS" CELL, vol. 79, 30 December 1994, pages 1157-1164, XP002071774 * the whole document * --- | 1-28 | |
| | -/-- | | |

EPO FORM 1503 03.82 (P04C10)

European Patent Office

# PARTIAL EUROPEAN SEARCH REPORT

Application Number
EP 98 10 6989

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.6) |
| D,A | PANDA, D. ET AL.: "Potential roles of osteopontin and alphaVbeta3 integrin in the development of coronary artery restenosis after angioplasty" PROCEEDINGS OF THE NATIONAL ACADEMY OF SCIENCES OF USA., vol. 94, August 1997, pages 9308-9313, XP002079003 WASHINGTON US * the whole document * --- | 1-28 | |
| D,A | PEYMAN A ET AL: "MINIMALLY MODIFIED OLIGONUCLEOTIDES-COMBINATION OF END-CAPPING AND PYRIMIDINE-PROTECTION" BIOLOGICAL CHEMISTRY HOPPE-SEYLER, vol. 377, no. 1, January 1996, pages 67-70, XP002041771 * the whole document * --- | 13-16 | TECHNICAL FIELDS SEARCHED (Int.Cl.6) |
| A | ROZZO C ET AL: "Induction of apoptosis in human neuroblastoma cells by abrogation of integrin -mediated cell adhesion." INTERNATIONAL JOURNAL OF CANCER, (1997 MAR 17) 70 (6) 688-98., XP002079004 * the whole document * --- | 1 | |
| T | TREASURYWALA, S. & BERENS, M.: "Migration arrest in glioma cells is dependent on the alphaV integrin subunit" GLIA, vol. 24, October 1998, pages 236-243, XP002079005 * the whole document * ----- | 1-28 | |

EPO FORM 1503 03.82 (P04C10)